# EUROPEAN PATENT SPECIFICATION

(11) **EP 3 615 563 B1**
(45) Date of publication and mention of the grant of the patent: **23.09.2020**
(21) Application number: 18722425.8
(22) Date of filing: 24.04.2018
(51) Int. Cl.: C07K 14/705, A61K 51/08, C07K 14/085, A61K 47/50, C07K 14/005, A61K 47/64, A61K 49/00

(54) **TUMOUR-TARGETING PEPTIDE VARIANTS**
AUF TUMORE ZIELENDE PEPTIDVARIANTEN
VARIANTES DE PEPTIDES CIBLANT LES TUMEURS

(30) Priority: 24.04.2017 GB 201706472
(43) Date of publication of application: 04.03.2020
(73) Proprietor: Cancer Research Technology Limited, London, Greater London EC1V 4AD (GB)
(72) Inventor: MARSHALL, John, London Greater London EC1V 4AD (GB); BRIMBLE, Margaret, Auckland 1142 (NZ)
(74) Representative: Mewburn Ellis LLP
(86) International application number: PCT/EP2018/060474
(87) International publication number: WO 2018/197490

(56) References cited:
- WO-A1-2015/160770
- WO-A2-2007/039728
- A. E. JOHN ET AL: "Preclinical SPECT/CT Imaging of alphavbeta6 Integrins for Molecular Stratification of Idiopathic Pulmonary Fibrosis", THE JOURNAL OF NUCLEAR MEDICINE, vol. 54, no. 12, 28 October 2013 (2013-10-28), pages 2146-2152, XP055483058, US ISSN: 0161-5505, DOI: 10.2967/jnumed.113.120592
- S. H. HAUSNER ET AL: "Targeted In vivo Imaging of Integrin alphavbeta6 with an Improved Radiotracer and Its Relevance in a Pancreatic Tumor Model", CANCER RESEARCH, vol. 69, no. 14, 23 June 2009 (2009-06-23) , pages 5843-5850, XP055231867, US ISSN: 0008-5472, DOI: 10.1158/0008-5472.CAN-08-4410
- SVEN H HAUSNER ET AL: "Use of a Peptide Derived from Foot-and-Mouth Disease Virus for the Noninvasive Imaging of Human Cancer: Generation and Evaluation of 4-[18F]Fluorobenzoyl A20FMDV2 for In vivo Imaging of Integrin alphavbeta6 Expression with Positron Emission Tomography", CANCER RESEARCH, AMERICAN ASSOCIATION FOR CANCER RESEARCH, US, vol. 67, no. 16, 15 August 2007 (2007-08-15), pages 7833-7840, XP002630513, ISSN: 0008-5472, DOI: 10.1158/0008-5472.CAN-07-1026

## Description

### Field of the invention

The present invention relates to peptide variants, conjugates and pharmaceutical compositions thereof and to their use in medicine, including for the treatment of cancer and imaging of tumours.

### Background to the invention

Integrins are αβ heterodimeric molecules that encompass a large family of cell surface receptors involved in several key processes including cell adhesion, invasion, proliferation and apoptosis [23]. In humans there are 24 members of which 8 classes recognize substrates via a highly conserved tripeptide motif Arg-Gly-Asp, present on extracellular ligands such as fibronectin and vitronectin [27]. Integrin αvβ6 is expressed in high levels on numerous cancers [4-9] such as oral, head and neck, pancreatic, ovarian and breast and increased expression level of integrin αvβ6 has been correlated with tumour progression. Integrin αvβ6 also can promote fibrosis [11] and some viral infections including foot and mouth disease [28]. As such, integrin αvβ6 is a major target [29-30] for imaging, diagnostics and therapy in the field of oncology and beyond.

A20FMDV2 (H₂N-¹NAVPNLRGDLQVLAQKVAR²⁰T-OH) (SEQ ID NO: 1) is a 20-residue linear peptide derived from the viral protein of foot-and-mouth disease virus [1-3, 10]. This peptide has been shown to exhibit high selectively and affinity for αvβ6, an integrin transmembrane receptor that is highly expressed in cancer cells [4-9]. A20FMDV2 binds to αvβ6 through the RGD tripeptide of the ⁷RGDLQV¹³L (SEQ ID NO: 2) fragment, and the two leucine residues present in the RGDLQVL fragment enhance the binding of the peptide to the receptor via hydrophobic interactions [10] and it is also stabilised by an α-helix generated from the C-terminal peptide motif ¹⁰Leu-²⁰Thr [1]. In addition, A20FMDV2 is an effective imaging agent for αvβ6 in pulmonary fibrosis when coupled with radioactive Indium (¹¹¹In) [24]. The [18F]fluorobenzoyl-labelled peptide, [18F]FBA-A20FMDV2, has found use as a PET radiotracer in human clinical trials [25].

A20FMDV2 has been used in positron emission tomography (PET) for diagnostic imaging applications [31] by conjugating to 4-[¹⁸F]fluorobenzoic acid (FBA) (or derivatives) [32-34], and ⁶⁴Cu labelling using A20FMDV2 peptide that incorporates a metal chelator such as DOTA [35-37]. Recently, [¹⁸F]-FBA-A20FMDV2 has progressed to a clinical setting [25] in the treatment regime of idiopathic pulmonary fibrosis [24] while an ¹¹¹In labelled A20FMDV2 derivative has been shown to be highly specific for imaging of breast cancer expressing the αvβ6 integrin using single-photon emission computed tomography (SPECT) [9]. These studies outline the importance of A20FMDV2 to ongoing diagnostic tools targeting the αvβ6 integrin and underpins αvβ6 as a valid therapeutic target.

WO2007/039723 describes αvβ6 peptide ligands, functional variants thereof and nucleic acids encoding them, as well as their use in the treatment and imaging of αvβ6 mediated diseases, including cancer.

Unfortunately, the therapeutic value of A20FMDV2 is limited by its short half-life in blood caused, in part, by it high susceptibility to serum proteases, such as endo- and exo-peptidases, as determined in mouse plasma studies [13].

One reported method for improving half-lives of susceptible peptides (and proteins) is the introduction of polyethyleneglycol (PEG) [38]. Hausner et *al.* prepared PEGylated versions of 4-[¹⁸F]-FBA-A20FMDV2, by placement of one or two PEG₂₈ moieties at the N terminus [13] or a single PEG₂₈ at both the N and C termini [39] and concluded that a bi-terminally PEGylated A20FMDV2 derivative, [¹⁸F]-FBA-PEG₂₈-A20FMDV2-PEG₂₈ exhibited the most favorable pharmacokinetics. Head-to-tail cyclisation of linear peptides is also a well-established method to minimise degradation by exopeptidases [40]; however, careful experimentation is required to prevent undesired side reactions such as polymerisation or racemisation.

However, there remains an unmet need for tumour-targeting peptides with potent binding activity, cellular uptake and extended plasma half-lives. The present invention addresses these and other needs.

### Brief description of the invention

Broadly, the present invention relates to A20FMDV2 peptide variants that have unexpectedly been found to exhibit enhanced αvβ6 binding activity in cell studies, and in some cases, enhanced cellular uptake as compared with A20FMDV2 peptide. In particular, as described in further detail herein, such peptide variants have been found to exhibit enhanced bio-distribution into αvβ6-expressing xenograft tumours (relative to control xenograft tumours lacking αvβ6 expression) as compared with a control A20FMDV2 peptide, as assessed by ¹¹¹In-labelled versions of the respective peptides. Such peptide variants therefore have great potential as tumour-targeted chemotherapeutic delivery agents and tumour imaging agents.

Accordingly, in a first aspect the present invention provides a peptide that selectively binds αvβ6 integrin, the peptide having an amino acid sequence comprising the motif X₁BₙRGDLX₂X₃X₄ZₘX₅ (SEQ ID D-Asn NO: 3), wherein X₁ is D-Asn Bn is a sequence of any n amino acids, which may be natural or unnatural, D- or L-, and may be the same or different, wherein n is a number between 1 and 10, X₂ and X₃ are independently selected from any amino acid, X₄ is Leu or Ile, Zₘ is a sequence of any m amino acids, which may be natural or unnatural, D- or L-, and may be the same or different, wherein m is a number between 1 and 10, X₅ is any L- or D-amino acid.

In some embodiments the length of the peptide is at least 17, 18, 19 or 20 amino acids. In some embodiments the length of the peptide is not more than 30, 29, 28, 27, 26, 25, 24, 23, 22, 21 or not more than 20 amino acids. In certain embodiments the length of the peptide is between 17 and 25 amino acids, e.g. between 19 and 21 amino acids. In particular embodiments the length of the peptide is 20 amino acids.

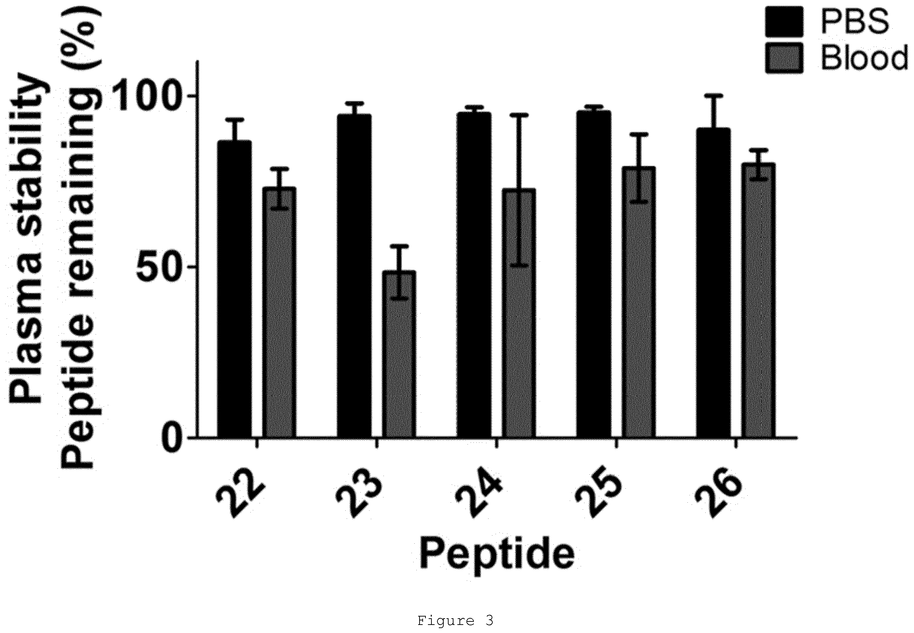

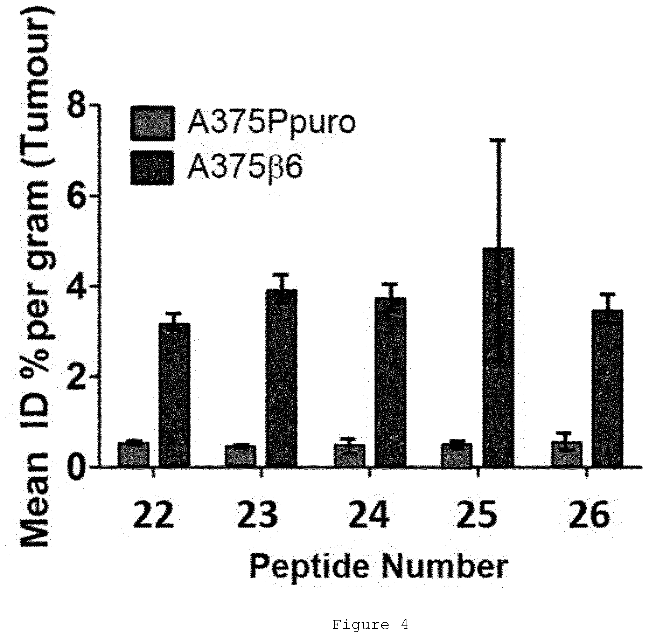

In the present invention X₁ is D-Asn. As shown in Tables 2 and 3 and Figures 3 and 4 herein, exemplary peptides wherein X₁ is D-Asn (peptides 19, 21, 25 and 26) exhibited superior relative activity (Table 2), high bio-distribution into an αvβ6-expressing tumour (Table 3 and Figure 4) and good plasma stability (Figure 3).

In some cases X₅ is L-Thr or D-Thr.

In some cases X₄ is L-Leu.

In some cases n is 5. In some cases m is 6. In certain cases n is 5 and m is 6.

In some cases Bₙ is AVPNL (SEQ ID NO: 4), KVPNL (SEQ ID NO: 5) or K(biotin)VPNL. Where Bₘ is K(biotin)VPNL, the biotin may be D-biotin attached to the Lysine side chain.

In some cases Zₘ is AQKVAR (SEQ ID NO: 6).

In certain cases the amino acid sequence of the peptide is selected from the group consisting of:
H₂N-[D-Asn]-AVPNLRGDLQVLAQKVART-COOH (SEQ ID NO: 7);
H₂N-[D-Asn]-KVPNLRGDLQVLAQKVART-COOH (SEQ ID NO: 8);
H₂N-[D-Asn]-K(biotin)VPNLRGDLQVLAQKVART-COOH (SEQ ID NO: 8);
H₂N-[D-Asn]-KVPNLRGDLQVLAQKVAR[D-Thr]-COOH (SEQ ID NO: 9);
H₂N-[D-Asn]-K(biotin)VPNLRGDLQVLAQKVAR[D-Thr]-COOH (SEQ ID NO: 9); and
H₂N-[D-Asn]-AVPNLRGDLQVLAQKVAR[D-Thr]-COOH (SEQ ID NO: 10).

In particular, the peptide may be:
H₂N-[D-Asn]-AVPNLRGDLQVLAQKVART-COOH (SEQ ID NO: 7).

In certain cases, the peptide may be N- and/or C-terminally modified. For example, the peptide may be N-acetylated and/or C-amidated.

In a second aspect, the present invention provides a conjugate comprising a peptide of the first aspect of the invention conjugated directly, or via a linker, to a therapeutic moiety, a polymer, a polypeptide and/or a detectable moiety.

In some embodiments, the peptide is conjugated to a therapeutic moiety which comprises an anti-cancer agent.

In some embodiments, the anti-cancer agent is selected from the group consisting of: an auristatin, a maytansinoids, a tubulysin, duocarmycin, calicheamicin, alpha-amanitin, a pyrrolobenzodiazepine (PBD), irinotecan, and an indolecarboxamide, or an analogue, derivative, prodrug or active metabolite thereof. In particular, the anti-cancer agent may comprise: Monomethyl Auristatin E (MMAE), mertansine (DM1), ravtansine (DM4) or Centanamycin.

In some cases the therapeutic moiety may comprise a therapeutic isotope. For example, therapeutic moiety may comprise an isotope selected from the group consisting of: ¹⁷⁷Lu, ⁹⁰Y, ²¹¹At, ²¹³Bi, ²¹²Pb, ²²⁵Ac, ²²³Ra, ⁴⁴Sc, ⁶⁷Ga, ¹³¹I, ¹⁸⁸Re, ¹⁸⁶Re and ⁶⁷Cu.

In some cases peptide is conjugated to a detectable moiety which is detectable by Magnetic Resonance Imaging (MRI), Magnetic Resonance Spectroscopy (MRS), Single Photon Emission Computed Tomography (SPECT), Positron Emission Tomography (PET) or optical imaging. In particular, the detectable moiety may comprise a fluorophore, a radionuclide or a spin label. In certain cases, the detectable moiety may comprise: ⁶⁸Ga, IRDye® 700, IRDye® 800, Fluorescein isothiocyanate (FITC), ⁸⁹Zr, ¹²⁴I, ⁶⁴Cu, ⁶²Cu, ¹⁸F, ⁸⁶Y, ¹¹¹In, 131_{I}, ¹²³I, ⁶⁷Ga or ^{99m}Tc.

In particular, the detectable moiety may comprise ¹¹¹In coupled to the peptide via a linker that comprises a chelate. In particular, the chelate may be selected from the group consisting of: diethylenetriamine pentaacetic acid (DTPA), tetrazacyclododecane-1,4,7,10-tetraacetic acid (DOTA), ethylenediaminetetraacetic acid (EDTA). The chelate (e.g. DTPA, DOTA or EDTA) may be linked to the N-terminus or C-terminus of the peptide (e.g. it may be linked to to D-Asn at the N-terminus).

In some cases peptide is conjugated to a polymer that enhances plasma half-life and/or biodistribution. In particular, the peptide may be conjugated to one or more (e.g. one or two, such as at each of the N-terminus and C-terminus) polyethylene glycol (PEG) groups. In certain cases the peptide may be conjugated to a moiety comprising -(OCH₂CH₂)ₙ-, wherein n ≥ 1. In particular, n may be 1 to 100, 1 to 50, 10 to 40, or 20 to 30. For example, n may be 1 or 2. In certain cases, the peptide may have two ethylene glycol groups present at the N-terminus or the C-terminus.

In a third aspect the present invention provides a pharmaceutical composition comprising:
a peptide of the first aspect of the invention or a conjugate of the second aspect of the invention; and
a pharmaceutically acceptable carrier.

In a fourth aspect the present invention provides a peptide of the first aspect of the invention, a conjugate of the second aspect of the invention or a pharmaceutical composition of the third aspect of the invention for use in medicine.

In a fifth aspect the present invention provides a peptide of the first aspect of the invention, a conjugate of the second aspect of the invention or a pharmaceutical composition of the third aspect of the invention for use in a method of treatment of an αvβ6-expressing tumour in a mammalian subject. In some embodiments the tumour is a cervical tumour, a head or neck tumour, a breast tumour, a lung tumour, a skin tumour, a colon tumour, an ovarian tumour or a pancreatic tumour. In particular cases, the conjugate or pharmaceutical composition thereof is a conjugate that comprises one or more of said anti-cancer agents. In certain cases, the method of treatment may comprise a step of determining whether the tumour in the mammalian subject expresses αvβ6, wherein said treatment is administered if the tumour is determined to express αvβ6.

The present description provides a method of treating a mammalian subject having an αvβ6-expressing tumour, the method comprising administering a therapeutically effective amount of a peptide of the first aspect of the invention, a conjugate of the second aspect of the invention or a pharmaceutical composition of the third aspect of the invention to the subject in need thereof. In some embodiments the tumour is a cervical tumour, a head or neck tumour, a breast tumour, a lung tumour , a skin tumour, a colon tumour, an ovarian tumour or a pancreatic tumour. In particular cases, the conjugate or pharmaceutical composition thereof is a conjugate that comprises one or more of said anti-cancer agents. In certain cases, the method of treatment may comprise a step of determining whether the tumour in the mammalian subject expresses αvβ6, wherein said treatment is administered if the tumour is determined to express αvβ6.

The present description provides use of a peptide of the first aspect of the invention, a conjugate of the second aspect of the invention or a pharmaceutical composition of the third aspect of the invention in the preparation of a medicament for the treatment of an αvβ6-expressing tumour in a mammalian subject. In some embodiments, the tumour is a cervical tumour, a head or neck tumour, a breast tumour, a lung tumour, a skin tumour, a colon tumour, an ovarian tumour or a pancreatic tumour. In particular cases, the conjugate or pharmaceutical composition thereof is a conjugate that comprises one or more of said anti-cancer agents. In certain cases, the method of treatment may comprise a step of determining whether the tumour in the mammalian subject expresses αvβ6, wherein said treatment is administered if the tumour is determined to express αvβ6.

In accordance with the fourth, or fifth aspect of the present invention, the peptide, conjugate or pharmaceutical composition may be administered or for administration with another anti-cancer agent and/or with radiotherapy or surgery. In particular, combination therapy with a second chemotherapeutic agent (simultaneous, sequential, separate or concurrent) and/or with radiotherapy and/or pre- or post-operatively are specifically contemplated. Without wishing to be bound by any particular theory, the present inventors believe that combining the αvβ6-targeting peptide or conjugate-based therapy of the present invention with blockade of one or more receptor tyrosine kinases (RTKs) may enhance anti-cancer therapeutic effectiveness in comparison with monotherapy. Preliminary antibody studies indicate that αvβ6 regulates and may enhance RTK signalling. Moreover, targeting the RTKs (e.g. epidermal growth factor receptor (EGFR) or erbB-2 (also known as human epidermal growth factor receptor 2 or *HER2*/*neu)* in tumour models in combination with an anti-αvβ6 antibody enhances therapy over monotherapy. Accordingly, it is specifically contemplated herein that the αvβ6-targeting peptide or conjugate-based therapy of the present invention may be combined with, in particular, one or more of: Trastuzumab (Herceptin®), Gefitinib (Iressa®), Erlotinib (Tarceva®), Lapatinib (Tykerb®), Cetuximab (Erbitux®) and Panitumumab (Vectibix®).

The present description a method of imaging an αvβ6-expressing tumour in a mammalian subject comprising administering a conjugate of the second aspect of the invention or a composition thereof, said conjugate comprising said detectable moiety, to the tumour-bearing subject and detecting said detectable moiety thereby forming an image of said tumour. Typically the image will be formed on a display (e.g. a digital display screen), but direct imaging on or in the body of the subject is specifically contemplated herein. In some embodiments, the tumour is a cervical tumour, a head or neck tumour, a breast tumour, a lung tumour, a skin tumour, a colon tumour, an ovarian tumour or a pancreatic tumour.

In a sixth aspect the present invention provides a conjugate of the second aspect of the invention or a composition thereof, said conjugate comprising said detectable moiety, for use in an *in vivo* method of diagnosis of cancer in a mammalian subject, wherein the method comprises administering said conjugate or said composition thereof to the subject and detecting said detectable moiety, thereby diagnosing the presence of an αvβ6-expressing tumour in the subject. In some cases, detecting said detectable moiety comprises measuring the amount, concentration, level, intensity and/or location of said detectable moiety and correlating or interpreting the measurement (such as by comparing the measurement with a reference level, background or control) to determine whether or not the measurement indicates presence of an αvβ6-expressing tumour in the subject and/or at a particular location in the body of the subject. In some embodiments, the tumour is a cervical tumour, a head or neck tumour, a breast tumour, a lung tumour, a skin tumour, a colon tumour, an ovarian tumour or a pancreatic tumour.

The present description provides a method of cancer diagnosis comprising administering a conjugate of the second aspect of the invention or a composition thereof, said conjugate comprising said detectable moiety, to a mammalian subject suspected of having an αvβ6-expressing tumour and detecting said detectable moiety thereby diagnosing the presence of an αvβ6-expressing tumour in the subject. In some cases, detecting said detectable moiety comprises measuring the amount, concentration, level, intensity and/or location of said detectable moiety and correlating or interpreting the measurement (such as by comparing the measurement with a reference level, background or control) to determine whether or not the measurement indicates presence of an αvβ6-expressing tumour in the subject and/or at a particular location in the body of the subject. In some embodiments, the tumour is a cervical tumour, a head or neck tumour, a breast tumour, a lung tumour a skin tumour, a colon tumour, an ovarian tumour or a pancreatic tumour.

In accordance with the present invention, the subject may be a human, a companion animal (e.g. a dog or cat), a laboratory animal (e.g. a mouse, rat, rabbit, pig or non-human primate), a domestic or farm animal (e.g. a pig, cow, horse or sheep). Preferably, the subject is a human. The subject may, in certain cases, be a human suffering from, diagnosed with or suspected of having an αvβ6-expressing tumour.

The present invention includes the combination of the aspects and preferred features described except where such a combination is clearly impermissible or is stated to be expressly avoided. These and further aspects and embodiments of the invention are described in further detail below and with reference to the accompanying examples and figures.

### Brief description of the figures

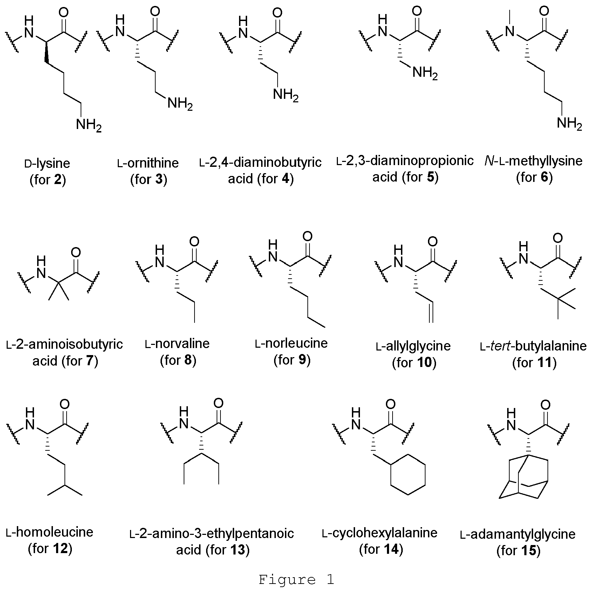

**Figure 1** shows the structures of the non-proteinogenic amino acids used for the synthesis of peptides **2-15.**

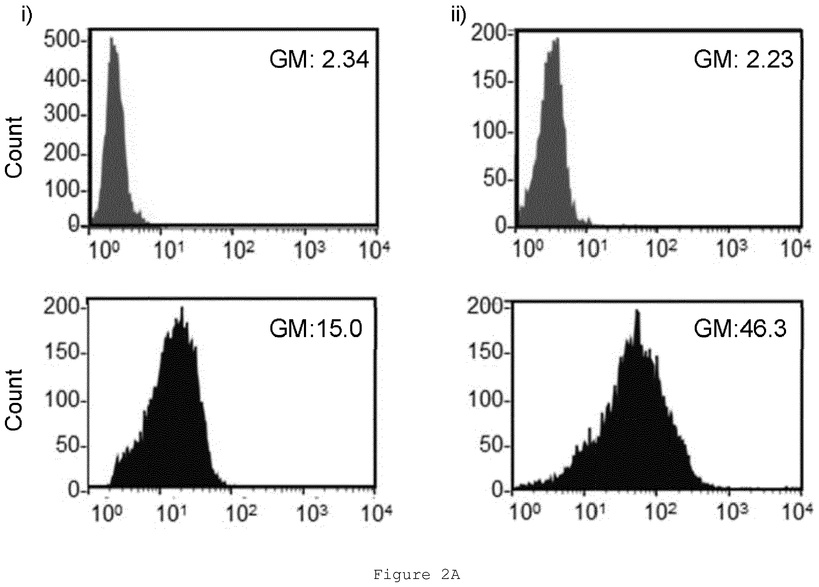

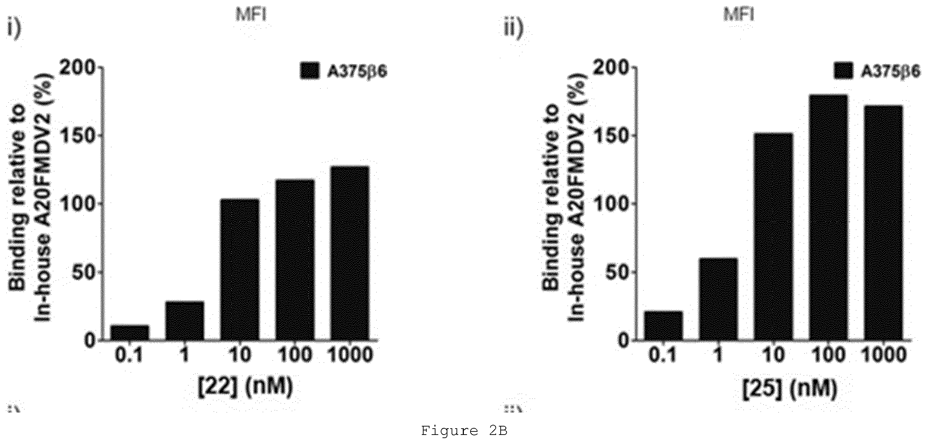

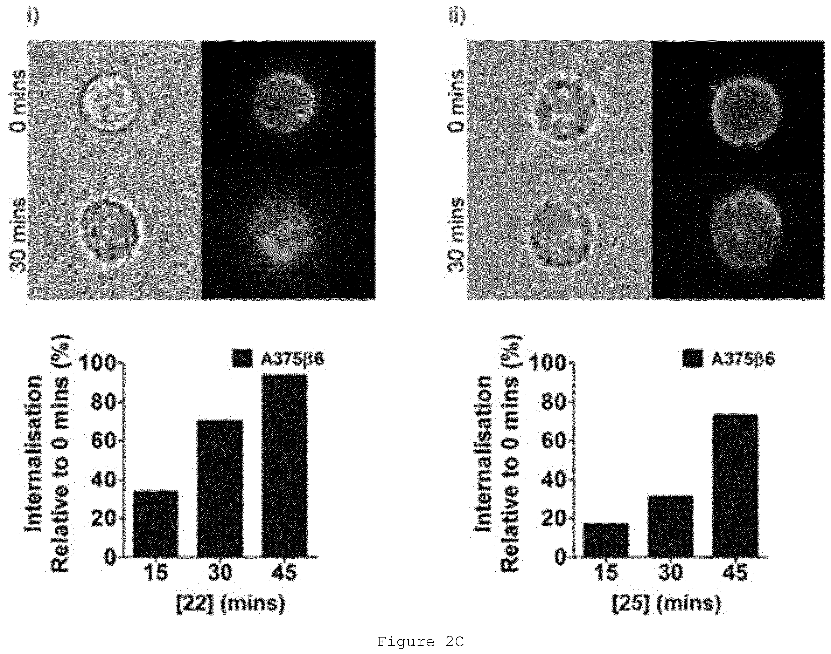

**Figure 2** shows a comparison of peptide **22** to peptide **25** testing *in vitro.* All A20FMDV2 variants were screened for specificity, activity and internalisation. **A**) Specificity is the absence of binding to A375Puro at 1000nM (upper histograms) and binding only to A375Pβ6 (lower histograms) and the shift of the histogram shows the Geometric Mean (GM) of the Mean Fluorescent Intensity (MFI) via flow cytometry at the same concentration of 100 nM shown by **Ai**) Peptide **22** (left-hand histograms) and **Aii**) peptide **25** (right-hand histograms). **B**) Affinity was determined by measuring the level of binding to A375Pβ6 at 0.1, 1, 10, 100 and 1000 nM via flow cytometry. The results show the binding of the peptides relative to the commercially sourced control biotinylated-A20FMDV2. Data from a single representative experiment shows that **Bii**) peptide **25** (right-hand graph) binds better to A375Pβ6 at similar concentrations compared to **Bi**) peptide **22** (left-hand graph). **C**) Internalisation shows αvβ6-dependent internalisation by A375Pβ6 cells using Imagestream®. Data shows the peptide at the surface of the cell (green, right-hand side images in each panel) at 0 minutes and after 45 minutes **Ci**) peptide **22** and **Cii**) peptide **25** have been internalised and they have a similar rate of internalisation (see graphs below the images; peptide **22** left-hand graph, peptide **25** right-hand graph).
**Figure 3** shows the stability of peptides in human plasma (n = 2) and PBS (control) after 24 h. Peptide **22, 25** and **26** are over 75% stable in plasma, in comparison to the peptide **23** and **24** that are less than 50% stable. Peptide levels were quantified using liquid chromatography-mass spectrometry (LC-MS).the stability of peptides in human plasma (labelled "blood", right-hand bars for each peptide) and PBS (left-hand bars for each peptide) determined after 24 hours. In this experiment, PBS was used as a control.
**Figure 4** shows the uptake of ¹¹¹In-DTPA coupled peptides in positive αvβ6-expressing tumour and negative control tumour (A375Puro). The peptides are specifically taken into αvβ6-expressing tumour (blue; right-hand bars for each peptide) compared to the negative tumour (red; left-hand bars for each peptide). The relative uptake is shown by the ratio A375Puro:A375β6. The data show that **25** is almost 10 times more specific to αvβ6-expressing tumour compared to the negative tumour. The data are plotted as mean Injected Dose (ID) per gram of tissue ± Standard Error of the Mean (SEM) for each of peptides **22-26.**

### Detailed description of the invention

In describing the present invention, the following terms will be employed, and are intended to be defined as indicated below.

### Peptide variants and unnatural amino acids

As defined herein, the peptides of the present invention are variants of the parent A20FMDV2 peptide and may comprise one or more unnatural amino acids, provided that the peptide is as defined in the claims. Suitable unnatural amino acids include, for example, D-amino acids, ornithine, diaminobutyric acid ornithine, norleucine ornithine, pyriylalanine, thienylalanine, naphthylalanine, phenylglycine, alpha and alpha-disubstituted amino acids, N-alkyl amino acids, lactic acid, halide derivatives of natural amino acids, such as trifluorotyrosine, p-Cl-phenylalanine, p-Br-phenylalanine, p-I-phenylalanine, L-allyl-glycine, b-alanine, L-a-amino butyric acid, L-g-amino butyric acid, L-a-amino isobutyric acid, L-e-amino caproic acid, 7-amino heptanoic acid, L methionine sulfone, L-norleucine, L-norvaline, p-nitro-L-phenylalanine, L-hydroxyproline, L-thioproline, methyl derivatives of phenylalanine - such as 1-methyl-Phe, pentamethyl-Phe, L-Phe(4-amino), L-Tyr(methyl), L-Phe(4-isopropyl), L-Tic(1,2,3,4-tetrahydroisoquinoline-3-carboxyl acid), L-diaminopropionic acid and L-Phe(4-benzyl). The peptides may be further modified. For example, one or more amide bonds may be replaced by ester or alkyl backbone bonds. There may be N or C alkyl substituents, side chain modifications or constraints such as disulphide bridges, side chain amide or ester linkages.

The peptides of the present invention may include both modified peptides and synthetic peptide analogues. Peptides may be, for example, be modified to improve formulation and storage properties, or to protect labile peptide bonds by incorporating non-peptidic structures.

The peptides of the present invention may include an N-terminal and/or C-terminal modification. For example, N-terminal acetylation and/or C-terminal amidation.

Peptides of the present invention may be prepared using methods known in the art. For example, peptides may be produced by chemical synthesis, e.g. solid phase techniques and automated peptide synthesisers, or by recombinant means (using nucleic acids such as those described herein). For example, peptides may be synthesised using solid phase strategies on an automated multiple peptide synthesizer (Abimed AMS 422) using 9-fluorenylmethyloxycarbonyl (Fmoc) chemistry. The peptides can then be purified by reversed phase-HPLC and lyophilized. Particular synthesis methods for making peptides of the invention are described in the Examples below.

In some cases X₂ and X₃ are helix promoting residues. In some cases the Zₘ amino acids are helix promoting residues. As used herein, the helix promoting residues may be independently selected from the group consisting of Glu, Ala, Leu, Met, Gln, Lys, Arg, Val, Ile, Trp, Phe and Asp. The helix promoting residues may include one or more artificial or modified amino acids.

### Detectable moiety

As used herein, the peptide of the present invention may be conjugated to a detectable moiety. The term "detectable moiety" relates to a moiety which, when located at the target site following administration of the conjugates of the invention into a patient, may be detected, typically non-invasively from outside the body and the site of the target located. Thus, the conjugates of this embodiment of the invention are useful in imaging and diagnosis. Readily detectable moieties are entities that are detectable by imaging techniques such as Magnetic Resonance Imaging (MRI), Magnetic Resonance Spectroscopy (MRS), Single Photon Emission Computed Tomography (SPECT) and Positron Emission Tomography (PET) and optical imaging. Preferably, imaging moieties are stable, nontoxic entities that retain their properties under *in vitro* and *in vivo* conditions. Examples of such moieties include but are not limited to radioactive moieties, for example radioactive isotopes. Suitable radioactive atoms include indium-Ill, technetium-99m or iodine-123 for scintigraphic studies. Other readily detectable moieties include, for example, spin labels for MRI such as iodine-123, iodine-131, indium-Ill, fluorine-18, carbon-13, nitrogen-15, oxygen-17, gadolinium, manganese or iron and optical moieties which include Cy5.5 and quantum dots. Further examples of detectable moieties include ⁶⁸Ga, IRDye® 700, IRDye® 800, Fluorescein isothiocyanate (FITC), ⁸⁹Zr, ¹²⁴I, ⁶⁴Cu, ⁶²Cu, ¹⁸F, ⁸⁶Y, ¹¹¹In, 131_{I}, ¹²³I, ⁶⁷Ga and ^{99m}Tc.

The term "therapeutic moiety" encompasses a moiety having beneficial, prophylactic and/or therapeutic properties.

Cytotoxic chemotherapeutic agents are well known in the art and include anti-cancer agents such as:
Alkylating agents including nitrogen mustards such as mechlorethamine (HN2), cyclophosphamide, ifosfamide, melphalan (L-sarcolysin) and chlorambucil; 10 ethylenimines and methylmelamines such as hexamethylmelamine, thiotepa; alkyl sulphonates such as busulfan; nitrosoureas such as carmustine (BCNU), lomustine (CCNLJ), semustine (methyl-CCN-U) and streptozoein (streptozotocin); and
triazenes such as decarbazine (DTIC;
dimethyltriazenoimidazolecarboxamide);
Antimetabolites including folic acid analogues such as methotrexate (amethopterin); pyrimidine analogues such as fluorouracil (5-fluorouracil; 5-FU), floxuridine (fluorodeoxyuridine; FUdR) and cytarabine (cytosine arabinoside); and purine analogues and related inhibitors such as mercaptopurine (6-mercaptopurine; 6-MP), thioguanine (6-thioguanine; TG) and pentostatin (2'-deoxycofonnycin). Natural Products including vinca alkaloids such as vinblastine (VLB) and vincristine; epipodophyllotoxins such as etoposide and teniposide; antibiotics such as dactinomycin (actinomycin D), daunorabicin (daunomycin; rubidomycin), doxorubicin, bleomycin, plicamycin (mithramycin) and mitomycin (mitomycin Q; enzymes such as L-asparaginase; and biological response modifiers such as interferon alphenomes. Miscellaneous agents including platinum coordination complexes such as cisplatin (cis-DDP) and carboplatin; anthracenedione such as mitoxantrone and antbracycline; substituted urea such as hydroxyurea; methyl hydrazine derivative such as procarbazine (N- methylhydrazine, MIH); and adrenocortical suppressant such as mitotane (o, p'-DDD) and aminoglutethimide; taxol and analogues/derivatives; and hormone agonists/antagonists such as flutamide and tamoxifen.

In particular, the peptide of the invention may be conjugated to an anti-cancer agent selected from an auristatin, a maytansinoids, a tubulysin, duocarmycin, calicheamicin, alpha-amanitin, a pyrrolobenzodiazepine (PBD), irinotecan, and an indolecarboxamide, or an analogue, derivatives, prodrug or active metabolite thereof. Specific examples include: Monomethyl Auristatin E (MMAE), mertansine (DM1), ravtansine (DM4) and Centanamycin.

In certain cases, the anti-cancer agent may be a cytotoxic peptide or polypeptide moiety by which we include any moiety which leads to cell death.

Cytotoxic peptide and polypeptide moieties are well known in the art and include, for example, ricin, abrin, *Pseudomonas* exotoxin, tissue factor and the like.

The use of ricin as a cytotoxic agent is described in Burrows & Thorpe, P.N.A.S. USA 90: 8996-9000, 1993, and the use of tissue factor, which leads to localised blood clotting and infarction of a tumour, has been described by Ran et al, Cancer Res. 58: 4646-4653, 1998 and Huang et al, Science 275: 25 547-550, 1997. Tsai et al, Dis. Colon Rectum 38: 1067- 1074, 1995 describes the abrin A chain conjugated to a monoclonal antibody Other ribosome inactivating proteins are described as cytotoxic agents in WO 96/06641. *Pseudomonas* exotoxin may also be used as the cytotoxic polypeptide moiety (see, for example, Aiello et al, P.N.A.S. USA 92: 10457-10461, 1995.

Certain radioactive atoms may also be cytotoxic if delivered in sufficient doses. Thus, the anti-cancer agent may comprise a radioactive atom which, in use, delivers a sufficient quantity of radioactivity to the target site so as to be cytotoxic. Suitable radioactive atoms include phosphorus-32, iodine-125, iodine-131, indium-Ill, rhenium-186, rhenium- 188, astatine-211 or yttrium-90, or any other isotope which emits enough energy to destroy neighbouring cells, organelles or nucleic acid. In particular, the therapeutic radioactive isotope may be selected from the group consisting of: ¹⁷⁷Lu, ⁹⁰Y, ²¹¹At, ²¹³Bi, ²¹²Pb, ²²⁵Ac, ²²³Ra, ⁴⁴Sc, ⁶⁷Ga, ¹³¹I, ¹⁸⁸Re, ¹⁸⁶Re and ⁶⁷Cu. Preferably, the isotopes and density of radioactive atoms in the compound of the invention are such that a dose of more than 4000 cGy, and more preferably at least 6000, 8000 or 10000 cGy, is delivered to the target site and, preferably, to the cells at the target site and their organelles, particularly the nucleus. The radioactive atom may be attached to the binding moiety in known ways. For example, EDTA, DTPA, DOTA or another chelating agent may be attached to the peptide of the invention and used to attach metal radionuclides including ¹¹¹In, ⁶⁸Ga, ⁹⁰Y or radioactive atoms as described above. Tyrosine residues may introduced into the peptide of the invention and may be labelled with ¹²⁵I or ^{I}311.

Methods of conjugating peptides to therapeutic agents are well known in the art. These may include use of linkers, for example cleavable linkers.

The term "pharmaceutically acceptable carrier" generally includes components that are compatible with the peptide or conjugate thereof and are not deleterious to the recipients thereof. Typically, the carriers will be water or saline which will be sterile and pyrogen free; however, other acceptable carriers may be used. Typically the pharmaceutical compositions or formulations of the invention are for parenteral administration, more particularly for intravenous administration.

The medicament or pharmaceutical composition of the present invention as defined above may usefully be administered to a patient who is also administered other medicaments, as it will be known to those skilled in the art. For example, in the case of cancer, the medicament or pharmaceutical composition of the present invention may be administered to a patient before, after or during administration of the other anti-tumour agent(s), for example before, after or during chemotherapy. Treatment with the peptide after chemotherapy may be particularly useful in reducing or preventing recurrence of the tumour or metastasis. For example, the anti-tumour agent can be covalently linked directly or indirectly to a peptide of the invention.

### Determining αvβ6 expression

The skilled person will be aware of numerous techniques for determining whether a sample (such as a tumour sample) expresses the integrin αvβ6. In particular embodiments, the subject to be treated may have a tumour having one or more cells that express the integrin αvβ6. In certain cases, the finding that the tumour expresses the integrin αvβ6 (i.e. that it is an αvβ6-positive cancer) may have been carried out previously or may be inferred from the type of cancer. For example, a number of tumour types are known to express the integrin αvβ6, including a cervical tumour, a head or neck tumour, a breast tumour, a lung tumour, a skin tumour, a colon tumour, an ovarian tumour or a pancreatic tumour. Without wishing to be bound by any particular theory, the present inventors believe cancers of the gastrointestinal (GI) tract and carcinomas that develop from many epithelial tissues may benefit from αvβ6-targeting therapy using a peptide or conjugate of the present invention. As the range of cancers found to express αvβ6 expands through additional research effort, so the range of cancers that may exhibit therapeutic benefit from αvβ6-targeting therapy using a peptide or conjugate of the present invention is expected to grow.

Additionally or alternatively, in certain cases the treatment may involve an active step of determining whether the tumour expresses the integrin αvβ6. Such a step may, for example, be carried out prior to initiating treatment with an αvβ6-targeting peptide or conjugate of the present invention in order to predict the likely suitability of the subject for therapy using an αvβ6-targeting peptide or conjugate of the present invention. Determining αvβ6 expression may involve measuring protein expression and/or level in a tumour sample using immunohistochemistry, measuring protein levels in a cell lysate by ELISA or Western blotting, and/or using a binding agent capable of specifically binding to αvβ6 or a fragment or subunit thereof.

In certain embodiments, determining whether the subject has an αvβ6-positive cancer may be performed on a nucleic acid extracted from a sample of cells obtained from the cancer, from a sample of cancer cells circulating in blood and/or from circulating tumour DNA (ctDNA) in blood or plasma.

In certain cases, determining the expression of αvβ6 comprises extracting RNA from a sample of cancer cells and measuring expression by real time PCR and/or by using a probe capable of hybridising to RNA encoding αvβ6 or a fragment thereof. The presence or amount of αvβ6 integrin may be determined directly using a binding agent, such as an antibody or a peptide or conjugate of the present invention, which is capable of specifically binding to αvβ6, or a fragment thereof. The binding agent may be labelled to enable it to be detected or capable of detection following reaction with one or more further species, for example using a secondary antibody that is labelled or capable of producing a detectable result, e.g. in an ELISA type assay.

### Samples

A "test sample" as used herein may be a cell or tissue sample (e.g. a biopsy), a biological fluid, an extract (e.g. a protein or DNA extract obtained from the subject). In particular, the sample may be a tumour sample, a blood sample (including plasma or serum sample), a cerebrospinal fluid sample, or a non-tumour tissue sample. The sample may be one which has been freshly obtained from the subject or may be one which has been processed and/or stored prior to making a determination (e.g. frozen, fixed or subjected to one or more purification, enrichment or extractions steps). Techniques for enriching a blood or plasma sample for circulating tumour DNA (e.g. based on fragment size) have been described. Moreover, sequencing techniques for identifying cancer-associated mutations in ctDNA have been described (e.g. based on digital PCR, targeted deep sequencing, nested real-time PCR, and the like).

"And/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.
It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

The following is presented by way of example and is not to be construed as a limitation to the scope of the claims.

### Examples

To enable binding activity to the αvβ6 integrin by flow cytometry to be measured, all peptides were synthesised incorporating a ²Lys(D-biotin) residue instead of native Ala (i.e. A20FMDV2, as disclosed in WO2007/039728, has Ala at position 2). For comparison we compared our own SPPS synthesized peptides with commercially synthesized biotinylated-A20FMDV2 (called biotinylated-A20FMDV2-A in Table 2 below). Following activity studies, biotinylated peptides exhibiting potent binding activity were then selected and the metal chelator diethylenetriamine pentaacetic acid (DTPA) [17] was incorporated to facilitate coupling of ¹¹¹In for plasma stability studies and *in vivo* biodistribution studies. The synthesis, binding studies and plasma stability of biotinylated A20FMDV2, its non-proteinogenic derivatives, and biotinylated and DPTA-incorporated analogues, are described in detail below.

### Experimental

### General information

All reagents were purchased as reagent grade and used without further purification. HPLC solvents were purchased as HPLC grade and used without further purification. FmocNH-L-Thr(^{t}Bu)-O-CH₂-phi-OCH₂-CH₂-CO₂H was purchased from PolyPeptide (Strasbourg, France). O-(Benzotriazol-1-yl)-*N,N,N',N*'-tetramethyluronium hexafluoro phosphate (HBTU), *O*-(7-azabenzotriazol-1-yl)-*N,N,N',N*'-tetramethyluronium hexafluorophosphate (HATU), Rink amide, 4-(hydroxymethyl)phenoxyacetic acid (HMP), Boc anhydride and *N,N-*diisopropylcarbodiimide (DIC) were purchased from GL Biochem (Shanghai, China). *N,N*-dimethylformamide (DMF) (AR grade) and acetonitrile (CH₃CN) (HPLC grade) were purchased from Scharlau (Barcelona, Spain). Diisopropylethylamine (*ⁱ*Pr₂NEt), 4-dimethylaminopyridine (DMAP), piperidine, *N*-methyl-2-pyrrolidone (NMP), collidine, 1,8-diazabicycloundec-7-ene (DBU), 2-mercaptoethanol, triisopropylsilane (TIPS) and 3,6-dioxa-1,8-octanedithiol (DODT) were purchased from Sigma-Aldrich (St Louis, MO). 2-Nitrobenzenesulfonyl chloride (2-NBS-Cl) and dimethylsulfate (DMS) were obtained from AK Scientific (Union City, CA). Trifluoroacetic acid (TFA) was purchased from Oakwood Chemicals (River Edge City, CA). The aminomethyl polystyrene resin and DTPA was synthesised according to published procedures [21, 22]. Fmoc-amino acids were purchased from GL Biochem with the following side chain protection: Fmoc-Arg(Pbf)-OH (Pbf = 2,2,4.6,7-pentamethyldihydrobenzofuran-5-sulfonyl), Fmoc-Asn(Trt)-OH (Trt = triphenylmethyl), Fmoc-Asp(^{t}Bu)-OH (^{t}Bu = *tert*-butyl), Fmoc-Gln(Trt)-OH, Fmoc-Glu (^{t}Bu) -OH, Fmoc-Lys(Boc)-OH, Fmoc-D-Lys(Boc)-OH, Fmoc-Orn (Boc) -OH, Fmoc-Dab(Boc)-OH, Fmoc-Dap(Boc)-OH, Fmoc-D-Thr (^{t}Bu) -OH and Fmoc-D-Asn(Trt)-OH.

Electrospray ionisation mass spectra (ESI-MS) were recorded on an Agilent Technologies (Santa Clara, CA) 1120 Compact LC connected to an in-line Hewlett Packard (Palo Alto, CA) 1100MSD spectrometer. Samples were introduced using direct flow injection at 0.2 mL/min into an ESI source in positive mode, using 0.1% formic acid/H₂O and 0.1% formic acid/CH₃CN (1:1, *v*/*v*)*.* Major and significant fragments were quoted in the form *x m*/*z* (mass to charge ratio). Analytical RP-HPLC was performed on an Ultimate 3000 system using a Waters XTerra® C18 column (5 µm, 4.6 x 150 mm) at a flow rate of 1 mL/min. A linear gradient of 0.1% TFA/H₂O (solvent A) and 0.1% TFA/CH₃CN (solvent B) was used with detection at 210 nm. Preparative RP-HPLC was performed on a Waters 600 System with a Waters 2487 dual wavelength absorbance detector using a Waters XTerra® Prep MS C18 column (10 µm, 19 x 300 mm) at a flow rate of 10 mL/min. Gradient systems were adjusted according to the elution profiles and peak profiles obtained from the analytical RP-HPLC chromatograms.

### General peptide synthesis procedure

Solid phase peptide synthesis (0.1 mmol scale) was performed on aminomethyl polystyrene resin (0.8 mmol/g) based on the Fmoc chemistry strategy. FmocNH-L-Thr(^{t}Bu)-O-CH₂-phi- OCH₂-CH₂-CO₂H was attached to the resin using general method A (see below), Rink amide was attached to the resin using general method B, and HMP was coupled to the resin using general method C [20]. Coupling of Fmoc-D-Thr(^{t}Bu)-OH was achieved using general method D. The desired peptide sequences were synthesised either manually or on a Tribute™ (Tucson, AZ) peptide synthesiser using general method E, peptide bond *N*-methylation was carried out using general method F, [19] and coupling of Fmoc-Gln(Trt)-OH to *N*-methylated lysine was carried out using general method G. N-Terminal acetylation was carried out using general method H, and tert-butyl protected DTPA was coupled to the peptides using general method I. D-Biotin was attached to the peptides using general method J. The linear peptides were cleaved from the resin using general method K, and the crude products were purified according to general method L.

### General method A: attachment of FmocNH-L-Thr(^{t}Bu)-O-CH₂-phi-OCH₂-CH₂-CO₂H to aminomethyl polystyrene resin

Aminomethyl polystyrene resin (0.1 mmol) was swollen in CH₂Cl₂/DMF (1:1, *v*/*v*) for 20 minutes and the solvent was removed by filtration. To the resin was added a solution of FmocNH-L-Thr(^{t}Bu)-O-CH₂-phi-OCH₂-CH₂-CO₂H (2 eq.) in 10% DMF/CH₂Cl₂ (1 mL, *v*/*v*) followed by DIC (2 eq.), and the reaction mixture was agitated at room temperature for 3 hours. The solution was drained, and the resin was washed with DMF (3 × 5 mL), CH₂Cl₂ (3 × 5 mL) and air dried to give **16.** A negative Kaiser test [41] indicated complete reaction.

### General method B: attachment of Rink amide to aminomethyl polystyrene resin

Aminomethyl polystyrene resin (0.1 mmol) was swollen in CH₂Cl₂/DMF (1:1, *v*/*v*) for 20 minutes and the solvent was removed by filtration. To the resin was added a solution of Rink amide (2 eq.) in 10% DMF/CH₂Cl₂ (1 mL, *v*/*v*) followed by DIC (2 eq.), and the reaction mixture was agitated at room temperature for 3 hours. The solution was drained, and the resin was washed with DMF (3 × 5 mL), CH₂Cl₂ (3 × 5 mL) and air dried. A negative Kaiser test indicated complete reaction.

### General method C: attachment of 4-(hydroxymethyl)phenoxyacetic acid (HMP) to aminomethyl polystyrene resin (synthesis of resin 27)

Aminomethyl polystyrene resin (0.1 mmol) was swollen in CH₂Cl₂/DMF (1:1, *v*/*v*) for 20 minutes and the solvent was removed by filtration. To the resin was added a solution of HMP (2 eq.) in 10% DMF/CH₂Cl₂ (1 mL, *v*/*v*) followed by DIC (2 eq.), and the reaction mixture was agitated at room temperature for 3 hours. The solution was drained, and the resin was washed with DMF (3 × 5 mL), CH₂Cl₂ (3 × 5 mL) and air dried to give ***27*.** A negative Kaiser test indicated complete reaction.

### General method D: attachment of Fmoc-D-Thr (^{t}Bu) -OH to HMP-resin 27

To the resin was added a solution of Fmoc-D-Thr(^{t}Bu)-OH (2 eq.) in DMF (1 mL) and DIC (2 eq.) followed by dropwise addition of DMAP (10% eq.) in DMF (100 µL). The reaction mixture was agitated at room temperature for 2 hours. After the solution was drained and the resin washed with DMF (3 × 5 mL), esterification was repeated for further 2 hours. To the resin was added 20% acetic anhydride/DMF (1 mL) and a solution of DMAP (10% eq.) in DMF (100 µL) dropwise, and the reaction mixture was agitated at room temperature for 1 hour after which the solution was drained and the resin washed with DMF (3 × 5 mL) .

### General method E: manually and automated Fmoc SPPS

To a solution of Fmoc protected amino acid (5 eq.) in DMF (1 mL) was added HBTU (4.75 eq.) and *ⁱ*Pr₂NEt (10 eq.), and the solution was added to the resin. After agitating the mixture at room temperature for 30 minutes, the solution was drained and the resin was washed with DMF (3 × 5 mL). The Fmoc protecting group was removed by treatment with 20% piperidine/DMF (5 mL, v/v) at room temperature for 5 minutes then 10 minutes.

### General method F: peptide bond N-methylation

*NBS protection:* To a solution of 2-NBS-Cl (4 eq.) in NMP (1 mL) was added collidine (10 eq.), and the solution was added to the resin and the reaction mixture agitated at room temperature for 15 minutes. After the solution was drained and the resin washed with NMP (3 × 5 mL), the reaction was repeated for further 10 minutes. *N-methylation:* To the resin was added a solution of DBU (3 eq.) in NMP (1 mL), and the reaction mixture was agitated at room temperature for 3 minutes. A solution of DMS (10 eq.) in NMP (1 mL) was then added to the reaction mixture, and the reaction mixture was agitated at room temperature for 2 minutes. After the solution was drained and the resin washed with NMP (3 × 5 mL), the reaction was repeated for further 2 minutes. *NBS deprotection:* To the resin was added a solution of 2-mercaptoethanol (10 eq.) and DBU (5 eq.) in NMP (2 mL), and the reaction mixture was agitated at room temperature for 5minutes. After the solution was drained and the resin washed with NMP (3 × 5 mL), the reaction was repeated for further 5 minutes.

### General method G: coupling of Fmoc-Gln(Trt)-OH to N-methylated Lys

To a solution of Fmoc-Gln(Trt)-OH (5 eq.) in DMF (1 mL) was added HATU (4.75 eq.) and *ⁱ*Pr₂NEt (10 eq.), and the solution was added to the resin and the reaction mixture agitated at room temperature for 3 hours. After the solution was drained and the resin washed with DMF (3 × 5 mL), the reaction was repeated for further 3 hours.

### General method H: N-terminal acetylation

To the resin was added a solution of 20% acetic anhydride/DMF (1 mL), followed by dropwise addition of a solution of DMAP (10% eq.) in DMF (100 µL). After the reaction mixture was agitated at room temperature for 30 minutes, the solution was drained, and the resin was washed with DMF (3 × 5 mL).

### General method I: attachment of tert-butyl protected DTPA

To a solution of **42** (5 eq.) in DMF (1 mL) was added HBTU (4.75 eq.) and *ⁱ*Pr₂NEt (10 eq.), and the solution was added to the resin. After agitating the mixture at room temperature for 30 minutes, the solution was drained and the resin was washed with DMF (3 × 5 mL).

### General method J: attachment of D-biotin

For the synthesis of peptides **1-15,** and peptides **17, 19, 20** and **21,** the *N*-terminal Fmoc group was removed by treatment with 20% piperidine/DMF (5 mL, *v*/*v*) at room temperature for 5 minutes then 10 minutes. After washing with DMF (3 × 5 mL), a solution of (Boc)₂O (5 eq.) in DMF (1 mL) was added to the resin, and the reaction mixture was agitated at room temperature for 30 minutes then washed with DMF (3 × 5 mL). The Dde group was removed by using 2% hydrazine/DMF (5 mL, *v*/*v*) at room temperature for 5 minutes and repeated with fresh reagents for 10 minutes. After washing with DMF (3 × 5 mL), a solution of D-biotin (5 eq.), HBTU (4.75 eq.) and *ⁱ*Pr₂NEt (10 eq.) in DMF (1 mL) was added to the resin, and the reaction mixture was agitated at room temperature for 30 minutes then washed with DMF (3 × 5 mL) .

For the synthesis of peptides **16** and **18** and peptides **22-26,** the Dde group was removed using 2% hydrazine/DMF (5 mL, v/v) at room temperature for 5 minutes and repeated with fresh reagents for 10 minutes. After washing with DMF (3 × 5 mL), a solution of D-biotin (5 eq.), HBTU (4.75 eq.) and *ⁱ*Pr₂NEt (10 eq.) in DMF (1 mL) was added to the resin, and the reaction mixture was agitated at room temperature for 30 minutes then washed with DMF (3 × 5 mL).

### General method K: cleavage of peptide from resin

The resin containing the desired linear peptide was agitated in a mixture of TFA/H₂O/DODT/TIPS (94:2.5:2.5:1.0, 5 mL, *v*/*v*/*v*/*v*) for 3 hours. To the filtrate was added cold diethyl ether (30 mL), and the product mixture was centrifuged for 10 minutes before the supernatant was discarded. This procedure was repeated two times. The resultant solid was dissolved in a solution of H₂O/CH₃CN+O.1% TFA (15 mL) and lyophilised.

### General method L: purification

The crude peptide was dissolved in a solution of H₂O/0.1%TFA and purified by preparative reverse-phase (RP)-HPLC on a XTerra^{®} C18 column using a gradient of 5-20%B at 0.5%B/min then 0.2%B/min up to 35%B, at a flow rate of 10 mL/min. The purity of the peptide was determined by flow injection (ESI⁺, 100V) and analytical RP-HPLC (XTerra® C18 column, 5-95%B, 3% B/min, 1 mL/min).

### Biology studies

### Preparation of peptide

Note that for comparison commercially sourced biotinylated DOTA-A20FMDV2 peptide, (95% purity) was purchased from PPR (Cambridge, UK). The peptides were re-suspended in 0.1%TFA (diluted in water) to prepare 1mM concentration of stock solutions which were aliquoted and stored at -20 °C.

### Cell lines

The cell lines used *in vitro* and *in vivo* are the transduced melanoma lines A375Ppuro, and A375Pβ6 (cultured in Dulbecco's Modified Eagle's Medium (DMEM)/10% fetal bovine serum (FBS) [9].

### Specificity and Level of Binding of peptides-Flow Cytometry method

Cells were trypsinised and re-suspended into DMEM/0.1% (w/v) sodium azide/0.1% (w/v) BSA (flow media) to prepare a cell suspension of 4 x10⁶ cells/ml. From this point on everything was kept on ice, 50µl of cell suspension was aliquoted into each flow tube (Falcon 2054 - Millipore). Peptides were serially diluted in the flow media at 2x concentration therefore when 50µl of each concentration was added to the 50µl cells it gave the final concentrations of 1000nM, 100nM, 10nM, 1nM, 0.1nM and 0nM. For A375Ppuro cells, only the top concentration of 1000nM was added to the cells. The peptides were then left on ice for 15 minutes. Cells were then washed twice by resuspension in flow media and centrifugation for 3 minutes at 1200rpm.

Mouse anti-biotin (50µl at 1:200; Jackson ImmunoResearch, 200-002-211) was added to each sample for 15 minutes, then wash steps were repeated. Goat anti-mouse IgG conjugated to Alexa 488 (1:250) was added (50µl) to each sample and left for 15 minutes on ice in the dark. The wash step was repeated and the cells were resuspended in 380µl of flow media. FACSCalibur cytometer (Becton-Dickinson) was used to acquire the data; geometric mean fluorescence intensity was recorded for each sample.

### Internalisation assay method - Imagestream and Flow Cytometry

Cells were trypsinised and resuspended into DMEM (serum-free media) to prepare a cell suspension of 4 x10⁶ cells/ml. From this point on everything was kept on ice. 50µl of cell suspension were aliquoted into each flow tube. Peptides were diluted in ice-cold serum-free media to 200nM giving the final concentration of 100nM when 50µl was added to the cell samples. The peptides were left on ice for 15 minutes then washed twice with flow media and centrifuged for 3 minutes at 1200rpm in between each wash step. Mouse anti-biotin (50µl at 1:200) was added to each sample for 15 minutes, then wash steps were repeated. Apart from the 0 time point, each of the time point samples were put in the incubator at 37°C and at 15, 30 and 45 minutes the cells were fixed in 4% formaldehyde in PBS for 10 minutes, washed once in PBS and 0.1% Triton X-100 detergent added for 5 minutes, before washing in PBS again. Goat anti-mouse IgG conjugated to AlexaFLUOR 488 (1:250) was added (50µl) to each sample and left for 15 minutes on ice in the dark. Samples were washed as above and resuspended to 30µl. Samples were examined on an Imagestream X Mark II (Amnis, Merck) Imagestream images were assessed for internalisation compared with the 0 minute samples using on board software.

### Internalisation assay method - Flow Cytometry

Cells were prepared as above except that no detergent was added prior to detecting the biotin with antibodies. Samples were analysed on a FACScalibur (Bekton-Dickinson) cytometer. Reduction in fluorescence signal measured over time was associated with internalization of bound peptide as confirmed visually using the Imagestream (data not shown).

### Plasma Stability

To 15 µg DTPA-conjugated peptide 15 MBq of Indium-111 (Covidien - Petten, Netherlands) was added (final volume 20µl) followed by 5µl Ammonium Acetate (1M). Samples were mixed and left for 30 minutes at room temperature. 2µl of this mixture was added to 100µl distilled water then the mixture injected into the gradient Reverse Phase-HPLC (Beckman). The sample was run through a Agilent Eclipse XDB-C18 Column in 0.1%TFA in distilled water. This was to check efficiency of radiolabelling which was typically > 95%. Human blood was taken in sodium heparin tubes and centrifuged at 2000g for 10mins. Plasma (300µl) was incubated with 7.5MBq of variant peptide and immediately 150µl was removed and placed at 37°C and the other 150µl was used as the time 0 minutes sample. To this was added and an equal volume of ice-cold Acetonitrile (1:1) and samples were mixed and centrifuged at 14000rpm for 5 minutes. The supernatants were collected and vacuumed dried for 10mins to remove acetonitrile. The residuum was filtered through a 0.22µm filter (to remove particulates) and 100µl was injected into the Radio-HPLC machine. The 24h samples were processed similarly. Control samples were incubated with PBS. (Note: PBS control did not require this process of filtration or addition of acetonitrile). Samples were performed in duplicate with similar results.

### Biodistribution

2x 10⁶ cells in 100µl were injected subcutaneously into the shoulders of female CD1 Nu/Nu mice. Tumours were grown subcutaneously on the left (A375Puro) and on the right (A375β6) shoulder over two weeks. Once tumours had grown to approximately 5mm diameter peptides were radiolabelled with Indium-111, and analysed by Radio-HPLC to determine labelling efficiency. The labelled peptides were diluted in sterile PBS/BSA and were dosed to give 0.3MBq per animal. Mice were then intravenously (tail-vein) injected with the radiolabelled peptide and are culled after 1 hour. Blood, organs and tumours were removed, weighed and residual activity was measured on a gamma counter (LKB Compugamma) the next day. Data were expressed as %Injected Dose/g tissue (%ID/g).

### Peptide Characterisation data

### Peptide H₂N-NK(Biotin)VPNLRGDLQVLAQKVART-OH (1) (SEQ ID NO: 11)

Peptide (**1**) (97.1 mg, 50%) was obtained as a white solid in >99% purity according to analytical RP-HPLC. *Rₜ* = 13.2 min (XTerra^{®} C18, 5-95% B, 3% B/min, 1.0 mL/min); *m*/*z* (ESI-MS): [M+2H]²⁺ Calc. 1224.3; Found 1223.6.

### Peptide H₂N-NK(Biotin)VPNLRGDLQVLAQkVART-OH (2) (SEQ ID NO: 12)

Peptide (**2**) (54.3 mg, 22%) was obtained as a white solid in >99% purity according to analytical RP-HPLC. *Rₜ* = 11.9 min (XTerra^{®} C18, 5-95% B, 3% B/min, 1.0 mL/min); *m*/*z* (ESI-MS): [M+2H]²⁺ Calc. 1224.3; Found 1223.7.

### Peptide H₂N-NK(Biotin)VPNLRGDLQVLAQ[ornithine]VART-OH (3) (SEQ ID NO: 13)

Peptide (**3**) (63.5 mg, 26%) was obtained as a white solid in >99% purity according to analytical RP-HPLC. *Rₜ* = 12.7 min (XTerra^{®} C18, 5-95% B, 3% B/min, 1.0 mL/min); *m*/*z* (ESI-MS): [M+2H]²⁺ Calc. 1217.3; Found 1216.7.

### Peptide N-NK(Biotin)VPNLRGDLQVLAQ[diaminobutyric acid]VART-OH (4) (SEQ ID NO: 14)

Peptide (**4**) (70.3 mg, 29%) was obtained as a white solid in >99% purity according to analytical RP-HPLC. *Rₜ* = 12.7 min (XTerra^{®} C18, 5-95% B, 3% B/min, 1.0 mL/min); *m*/*z* (ESI-MS): [M+2H]²⁺ Calc. 1210.3; Found 1209.8.

### Peptide H₂N-NK(Biotin) VPNLRGDLQVLAQ[diaminopropionic acid]VART-OH (5) (SEQ ID NO: 15)

Peptide (**5**) (59.9 mg, 25%) was obtained as a white solid in >99% purity according to analytical RP-HPLC. *Rₜ* = 12.9 min (XTerra^{®} C18, 5-95% B, 3% B/min, 1.0 mL/min); *m*/*z* (ESI-MS): [M+2H]²⁺ Calc. 1203.2; Found 1203.1.

### Peptide H₂N-NK(Biotin)VPNLRGDLQVLAQ-N-MeKVART-OH (6) (SEQ ID NO: 16)

Peptide (**6**) (72.7 mg, 30%) was obtained as a white solid in >99% purity according to analytical RP-HPLC. *Rₜ* = 11.5 min (XTerra^{®} C18, 5-95% B, 3% B/min, 1.0 mL/min); *m*/*z* (ESI-MS): [M+2H]²⁺ Calc. 1231.3; Found 1230.7.

### Peptide H₂N-NK(Biotin)VPNLRGDLQV[aminoisobutyric acid]AQKVART-OH (7) (SEQ ID NO: 17)

Peptide (**7**) (16.6 mg, 7%) was obtained as a white solid in >99% purity according to analytical RP-HPLC. *Rₜ* = 11.9 min (XTerra^{®} C18, 5-95% B, 3% B/min, 1.0 mL/min); *m*/*z* (ESI-MS): [M+2H]²⁺ Calc. 1209.7; Found 1210.2.

### Peptide H₂N-NK(Biotin)VPNLRGDLQV[norvaline]AQKVART-OH (8) (SEQ ID NO: 18)

Peptide (**8**) (19.5 mg, 8%) was obtained as a white solid in >99% purity according to analytical RP-HPLC. *Rₜ* = 12.2 min (XTerra^{®} C18, 5-95% B, 3% B/min, 1.0 mL/min); *m*/*z* (ESI-MS): [M+2H]²⁺ Calc. 1216.8; Found 1217.2.

### Peptide H₂N-NK(Biotin)VPNLRGDLQV[norleucine]AQKVART-OH (9) (SEQ ID NO: 19)

Peptide (**9**) (18.7 mg, 8%) was obtained as a white solid in >99% purity according to analytical RP-HPLC. *Rₜ* = 12.7 min (XTerra® C18, 5-95% B, 3% B/min, 1.0 mL/min); *m*/*z* (ESI-MS): [M+2H]²⁺ Calc. 1223.8; Found 1224.7.

### Peptide H₂N-NK(Biotin)VPNLRGDLQV[allylglycine]AQKVART-OH (10) (SEQ ID NO: 20)

Peptide (**10**) (5.5 mg, 2%) was obtained as a white solid in >99% purity according to analytical RP-HPLC. *Rₜ* = 11.7 min (XTerra^{®} C18, 5-95% B, 3% B/min, 1.0 mL/min); *m*/*z* (ESI-MS): [M+2H]²⁺ Calc. 1215.7; Found 1215.8.

### Peptide H₂N-NK(Biotin)VPNLRGDLQV[tert-butyl-alanine]AQKVART-OH (11) (SEQ ID NO: 21)

Peptide (**11**) (7.5 mg, 3%) was obtained as a white solid in >99% purity according to analytical RP-HPLC. *Rₜ* = 12.9 min (XTerra^{®} C18, 5-95% B, 3% B/min, 1.0 mL/min); *m*/*z* (ESI-MS): [M+2H]²⁺ Calc. 1230.8; Found 1230.7.

### Peptide H₂N-NK(Biotin)VPNLRGDLQV[homoleucine]AQKVART-OH (12) (SEQ ID NO: 22)

Peptide (**12**) (26.1 mg, 11%) was obtained as a white solid in >99% purity according to analytical RP-HPLC. *Rₜ* = 13.1 min (XTerra^{®} C18, 5-95% B, 3% B/min, 1.0 mL/min); *m*/*z* (ESI-MS): [M+2H]²⁺ Calc. 1230.8; Found 1231.2.

### Peptide H₂N-NK(Biotin)VPNLRGDLQV[2-amino-3-ethylpentanoic acid]AQKVART-OH (13) (SEQ ID NO: 23)

Peptide (**13**) (10.1 mg, 4%) was obtained as a white solid in >99% purity according to analytical RP-HPLC. *Rₜ* = 12.9 min (XTerra^{®} C18, 5-95% B, 3% B/min, 1.0 mL/min); *m*/*z* (ESI-MS): [M+2H]²⁺ Calc. 1230.8; Found 1230.8.

### Peptide H₂N-NK(Biotin)VPNLRGDLQV[cyclohexylalanine]AQKVART-OH (14) (SEQ ID NO: 24)

Peptide **(14)** (6.2 mg, 2%) was obtained as a white solid in >99% purity according to analytical RP-HPLC. *Rₜ* = 13.4 min (XTerra® C18, 5-95% B, 3% B/min, 1.0 mL/min); *m*/*z* (ESI-MS): [M+2H]²⁺ Calc. 1243.8; Found 1244.2.

### Peptide H₂N-NK(Biotin)VPNLRGDLQV[adamantylglycine]AQKVART-OH (15) (SEQ ID NO: 25)

Peptide (**15**) (22.4 mg, 9%) was obtained as a white solid in >99% purity according to analytical RP-HPLC. *Rₜ* = 13.7 min (XTerra^{®} C18, 5-95% B, 3% B/min, 1.0 mL/min); *m*/*z* (ESI-MS): [M+2H]²⁺ Calc. 1262.8; Found 1263.3.

### Peptide (16) Peptide AcHN-NK(Biotin)VPNLRGDLQVLAQKVART-OH (16) (SEQ ID NO: 11)

(with N-term. acetylation) (30.1 mg, 12%) was obtained as a white solid in >99% purity according to analytical RP-HPLC. *Rₜ* = 13.9 min (XTerra^{®} C18, 5-95% B, 3% B/min, 1.0 mL/min); *m*/*z* (ESI-MS): [M+2H]²⁺ Calc. 1244.8; Found 1245.2.

### Peptide H₂N-NK(Biotin)VPNLRGDLQVLAQKVART-NH₂ (17) (SEQ ID NO: 11) (with C-term. amidation)

Peptide (**17**) (13.4 mg, 5%) was obtained as a white solid in >95% purity according to analytical RP-HPLC. *Rₜ* = 13.5 min (XTerra^{®} C18, 5-95% B, 3% B/min, 1.0 mL/min); *m*/*z* (ESI-MS): [M+2H]²⁺ Calc. 1223.8; Found 1223.3.

### Peptide (18) Peptide AcHN-NK(Biotin)VPNLRGDLQVLAQKVART-NH₂ (18) (SEQ ID NO: 11)

(with N-term. acetylation and C-term. amidation) (12.5 mg, 5%) was obtained as a white solid in >95% purity according to analytical RP-HPLC. *Rₜ* = 13.9 min (XTerra^{®} C18, 5-95% B, 3% B/min, 1.0 mL/min); *m*/*z* (ESI-MS): [M+2H]²⁺ Calc. 1244.3; Found 1244.6.

### Peptide H₂N-nK(Biotin)VPNLRGDLQVLAQKVART-OH (19) (SEQ ID NO: 8)

Peptide (**19**) (47.7 mg, 19%) was obtained as a white solid in >95% purity according to analytical RP-HPLC. *Rₜ* = 12.6 min (XTerra^{®} C18, 5-95% B, 3% B/min, 1.0 mL/min); *m*/*z* (ESI-MS): [M+2H]²⁺ Calc. 1223.8; Found 1224.1.

### Peptide H₂N-NK(Biotin)VPNLRGDLQVLAQKVARt-OH (20) (SEQ ID NO: 26)

Peptide (**20**) (45.3 mg, 19%) was obtained as a white solid in >95% purity according to analytical RP-HPLC. *Rₜ* = 13.0 min (XTerra^{®} C18, 5-95% B, 3% B/min, 1.0 mL/min); *m*/*z* (ESI-MS): [M+2H]²⁺ Calc. 1223.8; Found 1224.2.

### Peptide H₂N-nK(Biotin)VPNLRGDLQVLAQKVARt-OH (21) (SEQ ID NO: 9)

Peptide (**21**) (36.4 mg, 15%) was obtained as a white solid in >95% purity according to analytical RP-HPLC. *Rₜ* = 12.9 min (XTerra^{®} C18, 5-95% B, 3% B/min, 1.0 mL/min); *m*/*z* (ESI-MS): [M+2H]²⁺ Calc. 1223.8; Found 1224.4.

### Peptide DTPA-NK(Biotin)VPNLRGDLQVLAQKVART-OH (22) (SEQ ID NO: 11) (N-term. DTPA)

Peptide (**22**) (53.7 mg, 24%) was obtained as a white solid in >99% purity according to analytical RP-HPLC. *Rₜ* = 13.3 min (XTerra^{®} C18, 5-95% B, 3% B/min, 1.0 mL/min); *m*/*z* (ESI-MS): [M+2H]²⁺ Calc. 1411.9; Found 1411.6.

### Peptide DTPA-nK(Biotin)VPNLRGDLQVLAQKVART-OH (25) (SEQ ID NO: 8) (N-term. DTPA)

Peptide (**25**) (37.5 mg, 13%) was obtained as a white solid in >95% purity according to analytical RP-HPLC. *Rₜ* = 12.6 min (XTerra^{®} C18, 5-95% B, 3% B/min, 1.0 mL/min); *m*/*z* (ESI-MS): [M+2H]²⁺ Calc. 1411.3; Found 1410.6.

### Peptide DTPA-nK(Biotin)VPNLRGDLQVLAQKVARt-OH (26) (SEQ ID NO: 9) (N-term. DTPA)

Peptide (**26**) (12.5 mg, 4%) was obtained as a white solid in >95% purity according to analytical RP-HPLC. *Rₜ* = 12.9 min (XTerra^{®} C18, 5-95% B, 3% B/min, 1.0 mL/min); *m*/*z* (ESI-MS): [M+2H]²⁺ Calc. 1411.3; Found 1410.6.

### Peptide DTPA-NK(Biotin)VPNLRGDLQVLAQKVART-NH₂ (24) (SEQ ID NO: 11) (N-term. DTPA and C-term. amidation)

Peptide (**24**) (12.1 mg, 4%) was obtained as a white solid in >95% purity according to analytical RP-HPLC. *Rₜ* = 13.0 min (XTerra^{®} C18, 5-95% B, 3% B/min, 1.0 mL/min); *m*/*z* (ESI-MS): [M+2H]²⁺ Calc. 1410.8; Found 1410.1.

### Peptide DTPA-GNK(Biotin)VPNLRGDLQVLAQKVART-OH (23) (SEQ ID NO: 27) (N-term. DTPA)

Peptide (**23**) (41.9 mg, 15%) was obtained as a white solid in >95% purity according to analytical RP-HPLC. *Rₜ* = 12.6 min (XTerra^{®} C18, 5-95% B, 3% B/min, 1.0 mL/min); *m*/*z* (ESI-MS): [M+2H]²⁺ Calc. 1439.9; Found 1439.2.

### Example 1 - Synthesis of biotinylated A20FMDV2 1, Lys16 or Leu13-modified and biotinylated peptides 2-15, N- and/or C-terminus-modified and biotinylated peptides 16-21 and (DTPA)-containing peptides 22-26.

To enable investigation of introducing non-proteinogenic amino acids (listed in Figure 1) on A20FMDV2 binding activity, biotinylated peptides **1-15** (see Table 1), except for peptide **6,** were synthesised by standard Fmoc SPPS on the acid liable hydroxymethylphenoxypropionic acid linker (HMPP) which delivers a C-terminal carboxylic acid using to the conditions depicted in Scheme 1. The desired peptide sequences were assembled using 20% piperidine/DMF to remove the Fmoc protecting group and *O-*(benzotriazol-1-yl)-*N,N,N',N*'-tetramethyluronium hexafluorophosphate (HBTU)/DIPEA as coupling reagents.

Since specific binding to the αvβ6 integrin was to be studied by flow cytometry, the native alanine at the second residue in A20FMDV2 (**1**) and all analogues thereof, were substituted with a biotinylated lysine residue. This substitution has previously been shown to be well tolerated [24,25]. We chose to install the D-biotin moiety by selective deprotection of a 1-(4,4-dimethyl-2,6-dioxocyclohex-1-ylidene)ethyl (Dde) [19] group on the side chain *Nε*-amino group followed by condensation with D-biotin using HBTU/DIPEA. Trifluoroacetic acid (TFA)/H₂O/3,6-dioxa-1,8-octanedithiol (DODT)/triisopropylsilane (TIPS) (94:2.5:2.5:1.0, v/v/v/v) effected cleavage of the synthesised peptides from the corresponding peptidyl-resins. Peptides **1-15** were obtained in good yields ranging from 2%-50% and purity exceeding 99% (see peptide characterization data).

For the synthesis of peptide **6** containing an N-L-methyllysine modification we employed an on-resin N-methylation protocol [22] which furnished peptide **6** in good yield (30%) following TFA-mediated peptide cleavage and RP-HPLC purification.

The lead peptide, A20FMDV2, which contains all naturally-occurring amino acids would be susceptible to degradation by exopeptidases which act on the amino- and carboxy terminuses. To mitigate this, six N- and/or C-terminus-modified and biotinylated A20FDMV2 mimics were prepared wherein we systematically modified the amino and carboxy ends (peptides **16-18)** and the N-terminal and C-terminal amino acids (Asn1 and Thr20, respectively, peptides **19-21).** N-terminal/C-terminal modified peptides **16-18** were obtained by capping of the N-terminus with acetic anhydride **(16)** or by employing the Rink amide linker to afford the C-terminal carboxamide **(17)** or a combination of both (peptide **18).**

Peptide **19,** bearing the unnatural D-Asn1 in place of the native Asn1 at the N-terminus of biotinylated A20FMDV2 (**1**) was obtained using the synthetic route outlined in Scheme 1 except that the Fmoc-D-Asn(Trt)-OH building block was incorporated into the synthesis as the N-terminal residue. For the preparation of peptides **20** and **21,** which contains the unnatural D-Thr at the C-terminus, HMP-anchored resin **27** (see Scheme 1, HMP = hydroxymethylphenoxyacetic acid) was first esterified with Fmoc-D-Thr(tBu)-OH using DIC/DMAP and the sequence then elongated by Fmoc SPPS.

**Table 1. List of prepared synthetic peptides [N-term]-X₁K(Biotin)VPNLRGDLQVX₂AQX₃VARX₄-[C-term] containing substitutions for the native Lys16 (peptides 2-6) or Leu13 (peptides 7-15), C-terminal/N-terminal variants (peptides 16-21) and DTPA-modified peptides (22-26). NB: nomenclature, particularly X position numbering used in this table is not the same as that used in the claims.**

| **Compound** | **N-term.** | **X₁** | **X₂** | **X₃** | **X₄** | **c-term.** |
|---|---|---|---|---|---|---|
| **1** | NH₂ | Asn | Leu | Lys | Thr | CO₂H |
| **2** | NH₂ | Asn | Leu | D-Lys | Thr | CO₂H |
| **3** | NH₂ | Asn | Leu | L-Orn | Thr | CO₂H |
| **4** | NH₂ | Asn | Leu | 1-2,4-diaminobutyric acid | Thr | CO₂H |
| **5** | NH₂ | Asn | Leu | 1-2,3-diaminopropionic acid | Thr | CO₂H |
| **6** | NH₂ | Asn | Leu | *N*-L-methyllysine | Thr | CO₂H |
| **7** | NH₂ | Asn | L-2-aminoisobutyric acid | Lys | Thr | CO₂H |
| **8** | NH₂ | Asn | L-norvaline | Lys | Thr | CO₂H |
| **9** | NH₂ | Asn | L-norleucine | Lys | Thr | CO₂H |
| **10** | NH₂ | Asn | L-allylglycine | Lys | Thr | CO₂H |
| **11** | NH₂ | Asn | L-tert-butylalanine | Lys | Thr | CO₂H |
| **12** | NH₂ | Asn | L-homoleucine | Lys | Thr | CO₂H |
| **13** | NH₂ | Asn | L-2-amino-3-ethylpentanoic acid | Lys | Thr | CO₂H |
| **14** | NH₂ | Asn | L-cyclohexylalanine | Lys | Thr | CO₂H |
| **15** | NH₂ | Asn | L-adamantylglycine | Lys | Thr | CO₂H |
| **16** | Ac-NH | Asn | Leu | Lys | Thr | CO₂H |
| **17** | NH₂ | Asn | Leu | Lys | Thr | CONH₂ |
| **18** | Ac-NH | Asn | Leu | Lys | Thr | CONH₂ |
| **19** | NH₂ | D-Asn | Leu | Lys | Thr | CO₂H |
| **20** | NH₂ | Asn | Leu | Lys | D-Thr | CO₂H |
| **21** | NH₂ | D-Asn | Leu | Lys | D-Thr | CO₂H |
| **22** | DTPA-NH | Asn | Leu | Lys | Thr | CO₂H |
| **23** | DTPA-Gly-NH | Asn | Leu | Lys | Thr | CO₂H |
| **24** | DTPA-NH | Asn | Leu | Lys | Thr | CONH₂ |
| **25** | DTPA-NH | D-Asn | Leu | Lys | Thr | CO₂H |
| **26** | DTPA-NH | D-Asn | Leu | Lys | D-Thr | CO₂H |

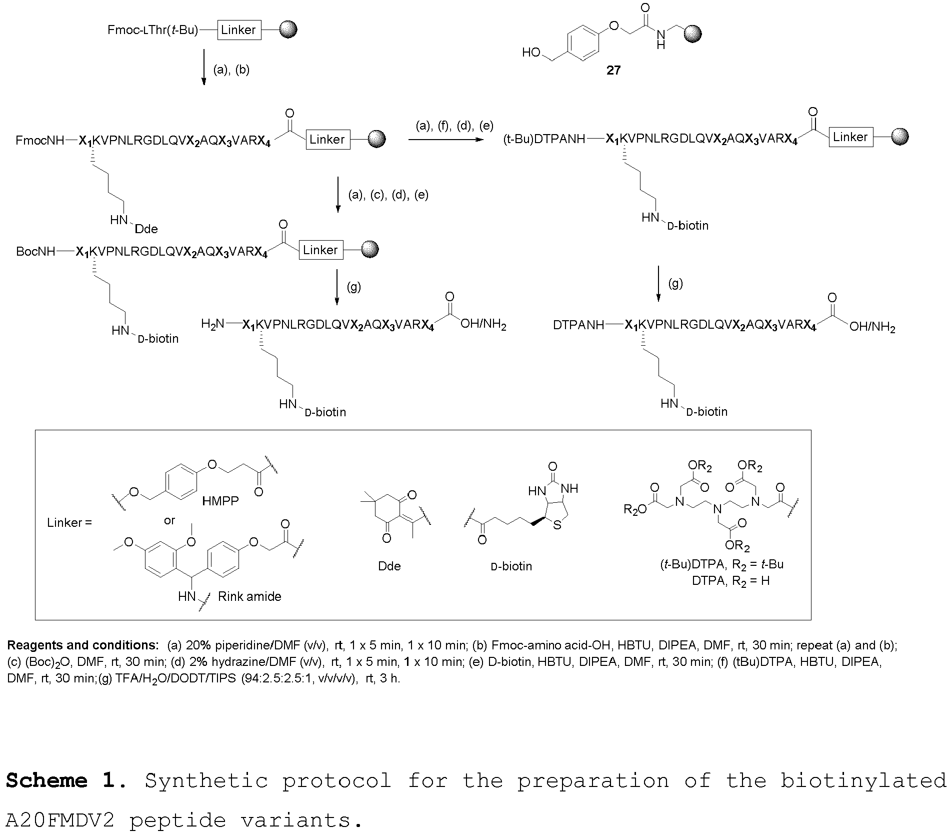

The results obtained from the binding assays (Table 2, see later) showed that modification of the N- and C-terminus of **1** (peptides **16** and **17** respectively) or substitution of the native Asn1, and both Asn1 and Thr20 residues of A20FMDV2 (**1**) for their D-counterparts (peptides **19** and **21,** respectively) resulted in peptides that exhibited improved biological activity compared to **1.** Four N- and C-termini-modified analogues thereof **(16, 18, 19** and **21)** were therefore selected for incorporation of the DTPA chelating agent to enable cell internalisation studies and degradation assays in human plasma to be carried out. The DTPA-containing, biotinylated A20FMDV2 peptide (**22**) was also prepared as a control [25].

As depicted in Scheme 1, the DTPA-containing, biotinylated A20FMDV2 peptide **22** and analogues **23-25)** were synthesised using Fmoc SPPS conditions (20% piperidine/DMF for Fmoc protecting group removal and HBTU/DIPEA as coupling reagent). To attach DTPA at the N-terminus of A20FMDV2 and D-biotin at the side chain of Lys2, the N-terminal Fmoc protecting group was first removed using 20% piperidine/DMF, and tert-butyl protected DTPA [21] was coupled to the resultant *Nα*-amino group using HBTU/DIPEA. As the four carboxylic acids of DTPA were protected as acid-labile tert-butyl esters, the Dde protecting group could be selectively removed using 2% hydrazine/DMF and D-biotin coupled to the side chain amino lysyl using HBTU/DIPEA. In the case of peptides **23** and **24** to enable the incorporation of the DTPA ligand a glycine spacer was employed to mimic the acetyl group of peptides **16** and **18** and to allow facile attachment of DTPA via amide bond formation.

### Example 2 - Biological activity of peptide variants

To test which, if any, of the peptide variants is superior to the original A20FMDV2 we designed a series of tests using two cell lines (A375Ppuro and A375Pβ6) that were genetically identical except for the expression of integrin αvβ6 in the A375Pβ6 cell lines [9]. Since A375Ppuro expresses four integrins (αvβ3, αvβ5, αvβ8 and α5β1) that also bind to RGD motifs in their ligands, comparing binding activity on these two cell lines is considered a highly stringent assay.

As an example of the assays used for characterizing the behaviour of modified peptides **1-15, 16-21** and **22-25,** Figure 2 shows a comparison between peptides **22** and **25.** A20FMDV2 binds to αvβ6 >1000-times more selectively than to other integrins [9]. To confirm that modified peptides retained specificity we determined how much peptide bound to A375Ppuro versus A375Pβ6 cells using flow cytometry. Figure 2A shows that at 1000 nM neither peptide **22** (Ai) nor peptide **25** (Aii) bound to A375Ppuro (red histograms; upper panels). In contrast both peptides bound well to A375Pβ6 (blue histograms; lower panels) at 100 nM. These data show that αvβ6 binding specificity is retained in these peptide variants. Moreover, every peptide variant failed to bind to A375Ppuro at 1000 nM (data not shown) showing that all peptides retained specificity.

To determine whether the modified peptides bound as well or better than the parent biotinylated-A20FMDV2 peptide at 100 nM we tested binding activity of variants at 0, 0.1, 1, 10, 100 and 1000 nM to A375Pβ6.

As an example, Figure 2B shows that peptide **22,** is better than the commercially obtained biotinylated-A20FMDV2 as it binds 16% higher at 100 nM. Similarly, peptide **25** is even better since at 100 nM binding is at 150% the commercially obtained biotinylated-A20FMDV2. These data were from single experiments. Table 2 shows the mean of up to four experiments for all peptides studied and shows overall, peptide **22** had on average 5% higher activity than the parent (i.e. commercially obtained biotinylated-A20FMDV2) and peptide **25** was 36% higher than the parent.

To assess ability to be internalized by cells at 37°C (and thus potential vectors for therapies) we used Imagestream® cytometry and flow cytometry. Imagestream® takes a fluorescence image of each cell (Figure 2C) enabling image analysis of the fluorescence on the surface versus the intracellular compartment. Histograms in Figure 2C plot internalization relative to the 0 minute time point. For flow cytometry we monitored the loss of surface expression as the peptides were internalized.

Table 2 summarizes the activity and internalization data for all peptides used in this study relative to the commercially sourced biotinylated-A20FMDV2 (the biotinylated A20FMDV2 (**1**), synthesized in this study, differs from the commercially obtained biotinylated-A20FMDV2 in that peptide (**1**) comprises the Ala2Lys mutation utilized to couple D-biotin).

Table 2 also provides an arbitrary value for each peptide's potential for intracellular drug delivery by multiplying binding affinity value by the amount internalized to give a Relative Activity value. It can be seen that most ¹⁶Lys and ¹³Leu modifications reduced relative activity; with the exception of peptides (**7**) and (**9**) their Relative Activity was lower than control biotinylated-A20FMDV2. In contrast most of the N- and C-terminus modified peptides **(16-19** and **21)** had better binding affinity and had improved Relative Activity compared to biotinylated-A20FMDV2 (**1**). For example, peptide **19** that has a non-proteinogenic D-Asn amino acid N-terminus modification bound on average 43% better to cellular αvβ6 than the parent compound (Table 2). Relative to unmodified, A20FMDV which has a value of 1.0 peptides **16** (N-acetylated), **17** (C-amidated), **18** (N-acetylated and C-amidated), **19** (D-Asn substitution) and **21** (both D-Asn and D-Thr substitution), have Relative Activity values of 55%, 31%, 9%, 27%, and 37% higher than the control peptide **1.**

**Table 2. Comparison of internalization data for Lys16 (1-6) or Leu13 (7-15) or C- or N-terminal modified (16-21) and DTPA-coupled (22-26)^{a} synthetic peptides.**

| **Compound** | **A) Affinity***^{b}* | **B) Internalisation^{c}** | **Relative Activity (AxB)** |
|---|---|---|---|
| ***A20FMDV2^{d}*** | 1.00 | 1.00 | 1.00 |
| **1^{e}** | 1.11±0.15 | 0.84±0.14 | 0.93 |
| **2** | 1.11±0.15 | 0.78±0.15 | 0.87 |
| **3** | 1.12±0.16 | 0.86±0.29 | 0.96 |
| **4** | 1.00±0.07 | 0.86±0.29 | 0.86 |
| **5** | 0.79±0.14 | 0.96±0.31 | 0.76 |
| **6** | 0.37±0.12 | 0.89±0.32 | 0.32 |
| **7** | 1.07±0.37 | 1.11±0.3 | 1.19 |
| **8** | 0.77±0.52 | 1.09±0.17 | 0.84 |
| **9** | 1.16±0.29 | 1.15±0.04 | 1.33 |
| **10** | 0.58±0.25 | 0.82±0.25 | 0.48 |
| **11** | 0.60±0.23 | 1.03±0.14 | 0.62 |
| **12** | 0.45±0.22 | 0.66±0.14 | 0.27 |
| **13** | 0.70±0.33 | 0.52±0.23 | 0.37 |
| **14** | 0.62±0.06 | 1.01±0.32 | 0.62 |
| **15** | 0.51±0.28 | 1.07±0.18 | 0.55 |
| **16** | 1.48±0.49 | 1.05±0.24 | 1.55 |
| **17** | 1.23±0.25 | 1.07±0.40 | 1.31 |
| **18** | 1.25±0.26 | 0.87±0.12 | 1.09 |
| **19** | 1.43±0.51 | 0.89±0.12 | 1.27 |
| **20** | 0.53±0.44 | 0.99±0.25 | 0.53 |
| **21** | 1.57±0.55 | 0.87±0.27 | 1.37 |
| **22** | 1.01±0.21 | 1.05±0.07 | 1.06 |
| **23** | 1.28±0.23 | 0.97±0.11 | 1.24 |
| **24** | 1.19±0.30 | 0.98±0.12 | 1.17 |
| **25** | 1.36±0.42 | 0.99±0.10 | 1.34 |
| **26** | 1.19±0.40 | 1.04±0.16 | 1.24 |

| | | | |
|---|---|---|---|
| *^{a}*Specific peptides (**1, 16, 17, 19, 21**) were DTPA- coupled. *^{b}*Mean activity relative to biotinylated **A20FMDV2** (100 nM). *^{c}*Mean % internalisation at 45 min relative to biotinylated **A20FMDV2** (100 nM) using flow cytometry. *^{d}*Biotinylated DOTA-A20FMDV2 obtained commercially. *^{e}*Biotinylated A20FMDV2 synthesised in this work. | | | |

Encouragingly, conjugation of DTPA to lead peptides **(16, 17, 19, 21)** to create peptides **23-26,** respectively, had very little effect on binding affinity or internalization and thus maintained their increased Relative Activity.

### Example 3 - Plasma stability and bio-distribution studies

Figure 3 shows the stability of DTPA-conjugated peptides **(22-26)** in plasma (red bars; right-hand bar for each peptide) versus PBS (black bars; left-hand bar for each peptide) at 37°C, relative to a 0 minutes time point. The data show that after 24 h at 37°C in PBS only about 10% of the peptide has degraded. In contrast, while peptides **(22, 25, 26)** show similar levels (77%-80%) remaining in plasma after 24 hours, there is major degradation of peptide (**23**) (60% remaining) and peptide (**24**) (52% remaining). Thus, the modifications of peptides **24** and **23** (C-terminal amidation and N-terminal acetylation, respectively) have increased susceptibility to plasma degradation relative to the control parent peptide **(1).** Peptide modification by chemical manipulation using D amino acids (D-Asn, compound **25,** and D-Asn/D-Thr compound **26**) was optimal in improving susceptibility to human plasma proteases.

Immunocompromised mice (n=3-5) bearing A375Ppuro and A375Pβ6 tumours on opposite shoulders were given intravenous (iv) injections of 300Bq of radiolabelled peptides **22-26** then killed 1 h later. Major organs and blood were collected immediately, weighed, and radioactivity determined. The data in Table 3 show the behaviour of the five DTPA-conjugated peptides tested; data are expressed as mean % injected dose/g tissue.

**Table 3. Bio-distribution (Mean (%) ID/g ± SEM) data for ¹¹¹In-DTPA labelled-peptides (22-26)**

| **Tissue** | **22** | **23** | **24** | **25** | **26** |
|---|---|---|---|---|---|
| Tumour (αvβ6)^{a} | 3.1 ± 0.18 | 3.7 ± 0.29 | 3.8 ± 0.31 | 4.8± 2.43 | 3.4 ± 0.21 |
| Tumour (Control)*^{a}* | 0.5 ± 0.04 | 0.5 ± 0.15 | 0.4 ± 0.04 | 0.5± 0.11 | 0.5 ± 0.24 |
| Intestines | 4.1 ± 0.23 | 3.8 ± 0.98 | 3.4 ± 0.29 | 4.2± 0.06 | 3.9 ± 0.22 |
| Pancreas | 0.5 ± 0.06 | 0.5 ± 0.13 | 0.5 ± 0.03 | 0.5± 0.03 | 0.5 ± 0.03 |
| Spleen | 0.1 ± 0.01 | 0.2 ± 0.07 | 0.2 ± 0.01z | 0.1± 0.01 | 0.19 ± 0.01 |
| Stomach | 6.5 ± 1.05 | 6.4 ± 2.33 | 4.6 ± 0.63 | 6.4± 0.97 | 6.6 ± 0.71 |
| Kidney | 85.9 ± 8 | 154.0 ± 19.44 | 131.2 ± 12 | 67.0 ± 6.65 | 62.0 ± 3.98 |
| Liver | 0.3 ± 0.02 | 0.4 ± 0.06 | 0.3 ± 0.05 | 0.3 ± 0.03 | 0.4 ± 0.03 |
| Heart | 0.6 ± 0.06 | 0.6 ± 0.16 | 0.66 ± 0.02 | 0.7 ±0.01 | 0.7 ± 0.07 |
| Lung | 2.7 ± 0.24 | 4.4 ± 0.72 | 3.62 ± 0.30 | 2.2 ± 0.22 | 2.5 ± 0.17 |
| Blood | 0.4 ± 0.03 | 0.5 ± 0.16 | 0.3 ± 0.04 | 0.4 ± 0.05 | 0.5 ± 0.01 |
| Muscle | 1.1 ± 0.10 | 0.8 ± 0.07 | 1.1 ± 0.05 | 0.9 ± 0.09 | 1.2 ± 0.1 |
| Tail | 2.1 ± 0.43 | 4.3 ± 2.85 | 1.0 ± 0.19 | 0.8 ± 0.04 | 1.3 ± 0.39 |

| | | | | | |
|---|---|---|---|---|---|
| *^{a}*Data are derived from excised tissues taken from mice (n = 3) bearing pairs of A375β6 (αvβ6) and A375Puro (control) xenografts. Mice were culled 1 h after iv injection of 100 Bq of individual peptides. Data are displayed as mean % injected dose per gram of tissue ± standard error of the mean. Bq: Becquerel. | | | | | |

As with our previous studies, there is significant retention in the gastrointestinal tract (stomach and intestines) and also the lungs [9, 24]. The kidney retention, which we reported is independent of αvβ6 [9], is highly varied for the five peptides with peptides **25** and **26** showing the least retention and peptides **23** and **24** showing retention far greater than peptide **22,** which is the parent biotinylated-A20FMDV2. There is a robust selective uptake of each peptide by the αvβ6-positive tumour compared with the αvβ6-negative control tumour. Figure 4 highlights these differences in a histogram. While there is no statistical increased uptake of the peptides **23-26** versus the DTPA-labelled parent peptide **22,** all the variants have slightly higher mean ratio of retention in the αvβ6-positive tumour verses αvβ6-negativetumour compared to the control peptide **22.** The highest ratio of retention in the αvβ6-positive tumour verses αvβ6-negative tumour compared to the control peptide **22** was seen with peptide **25.**

### Discussion

The 20-residue linear peptide A20FMDV2 exhibits high specificity and affinity for the tumour-related αvβ6 integrin, and also exhibits αvβ6-dependent internalisation into cells. A20FMDV2 is therefore considered a promising lead compound to deliver therapeutic loads (e.g. chemotherapeutic payloads) to αvβ6-expressing cancer cells. In the Examples above, the impacts of non-proteinogenic substitutes of the native amino acid residues of A20FMDV2 on *in vitro* behaviour, plasma stability and *in vivo* bio-distribution have been investigated.

All of the peptides described herein were synthesised in good yield and excellent purity by Fmoc SPPS. Binding studies to the αvβ6 integrin showed that all the A20FMDV2 analogues bound only to A375Pβ6 cells but not A375Ppuro cells showing that specificity had not been affected by modifications. Moreover, peptides incorporating an acetylated N-terminus and an amidated C-terminus or the unnatural D-version of the native ¹Asn and ²⁰Thr residues exhibited more potent αvβ6-binding activity than the parent peptide (A20FMDV2) and also were more potent than most peptides containing non-proteinogenic substitutes of the native ¹⁶Lys and ¹³Leu residues. For example, peptide **19** that has a non-proteinogenic D-Asn amino acid N-terminus modification bound on average 43% better to cellular αvβ6 than the parent compound (Table 2).

We have assessed stability in human blood plasma and showed that the peptides remained intact for much longer than in our previous studies in mouse serum which suggests that human plasma is less degradative than mouse serum for these peptides. While over 90% of all radiolabelled peptides remained intact in PBS after 24 h at 37 °C, only A20FMDV2 containing D amino acids **25** and **26** remained mostly intact (76-80%) after 24 h in human plasma at 37 °C.

The modifications had only moderate effects on the degree of internalisation into αvβ6-expressing cells. Thus, Table 2 shows that for all peptides tested, internalisation varied from 0.78 to 1.11 fold relative to commercial biotinylated-A20FMDV2. Since both the binding affinity and internalisation of the peptide is necessary to consider for delivery of an intracellular cytotoxic, Table 2 also shows an arbitrary value for each peptide's Relative Activity. The control peptide has a value of 1.0 whereas peptides **16, 19, 17, 18,** and **21,** have Relative Activity values of 55%, 27%, 31%, 9% and 37% higher than the control peptide, respectively.

For αvβ6-targeting peptides to deliver therapies they must be selectively retained in αvβ6-expressing tumours. In bio-distribution studies we found that all of the five lead peptides tested **(22-26)** were selectively retained in the αvβ6-positive tumours at least six-to ten-fold higher levels than the αvβ6-negative tumours. It is encouraging that all the modified peptides were slightly better than the DTPA-labelled and biotinylated peptide **22.** The present results show that chemical modification of A20FMDV2 has improved its capacity for selectively locating to αvβ6-expressing cancers.

### References

A number of publications are cited above in order to more fully describe and disclose the invention and the state of the art to which the invention pertains. Full citations for these references are provided below.
[1] DiCara et al., J. Biol. Chem., 2007, Vol. 282, pp. 9657-9665.
[2] Jackson et al., J. Virol., 2000, Vol. 74, pp. 4949-4956.
[3] Logan et al., Nature, 1993, Vol. 362, pp. 566-568.
[4] Bandyopadhyay et al., Curr. Drug Targets, 2009, Vol. 10, pp. 645-652.
[5] Hazelbag et al., J. Pathol., 2007, Vol. 212, pp. 316-324.
[6] Elayadi et al., J. Cancer Res., 2007, Vol 67, pp. 5889-5895.
[7] Thomas et al., J. Oral Pathol. & Med., 2006, Vol. 35, pp. 1-10.
[8] Bates et al., J. Clin. Invest., 2005, Vol. 115, pp. 339-347.
[9] Saha et al., J. Pathol., 2010, Vol. 222, pp. 52-63.
[10] DiCara et al., J. Virol., 2008, Vol. 82, pp. 1537-1546.
[11] Munger et al., Cell, 1999, Vol. 96, pp. 319-328.
[12] Firer et al., J. Heamatol. Oncol., 2012, Vol. 5, p. 70.
[13] Hausner et al., Cancer Res., 2009, Vol. 69, pp. 5843-5850.
[14] Gentilucci et al., Curr. Pharm. Des., 2010, Vol. 16, pp. 3185-3203.
[15] Galati et al., Naturforsch. Sect. C J. Biosci. 2003, Vol. 58, pp. 558-561.
[16] PeptideCutter. ExPASy Bioinformatics Resource Portal. Obtained from http://web.expasy.org/peptide_cutter/
[17] Safavy et al., J. Bioconjugate Chem., 2002, Vol. 13, pp. 317-326.
[18] Nash et al., Tetrahedron Lett., 1996, Vol. 37, pp. 2625-2628.
[19] Chatterjee et al., Nature Protoc., 2012, Vol. 7, pp. 432-444.
[20] Harris et al., Synthesis, 2009, Vol. 20, pp. 3460-3466.
[21] Arano et al., J. Med. Chem., 1996, Vol. 39, pp. 3451-3460.
[22] Harris et al., Tetrahedron Lett., 2011, Vol. 52, pp. 6024-6026.
[23] Hynes et al., Cell, 2002, Vol. 110, pp. 673-687.
[24] John et al., J. Nucl. Med., 2013, Vol. 54, pp. 2146-2152.
[25] Clinical Trial NCT02612051 https://clinicaltrials.gov/ct2/show/NCT02612051 (last accessed 31 January 2017).
[27] Ruoslahti et al., Annu. Rev. Cell Dev. Biol. 1996, Vol. 12, pp. 697-715.
[28] Zhang et al., Viruses, 2013, Vol. 5, pp. 1114-1130.
[29] Zhu et al., Mol. Pharmacueticals, 2014, Vol. 11, pp. 2108-1217.
[30] Maltsev et al. Angew. Chem. Int. Ed., 2016, Vol. 55, pp. 1535-1539.
[31] Liu et al., Am. J. Med. Mol. Imaging, 2014, Vol. 4, pp. 333-345.
[32] Hausner et al., Cancer Res., 2007, Vol 67, pp. 7833-7840.
[33] Hausner et al., J. Med. Chem., 2008, Vol. 51, pp. 5901-5904.
[34] Hausner et al., Nucl. Med. Biol., 2013, Vol. 40, pp. 233-239.
[35] Hausner et al., Mol. Imaging, 2009, Vol. 8, pp. 111-121.
[36] Hu et al., Mol. Imaging Biol., 2014, Vol. 16, pp. 567-577.
[37] Satpati et al., J. Radioanal. Nucl. Chem., 2014, Vol. 302, pp. 765-771.
[38] Hamley, I. W., Biomacromolecules, 2014, Vol. 15, 1543-1559.
[39] Hausner et al., J. Nucl. Med., 2015, Vol. 56, pp. 784-790.
[40] Demmer et al., Design of cyclic peptides. In Peptide and protein design for biopharmaceutical applications, Jensen, K. J., Ed. John Wiley & Sons Ltd: 2009; pp 133-166.
[41] Kaiser et al., Anal. Biochem. 1970, Vol. 34, pp. 595-598.

The specific embodiments described herein are offered by way of example, not by way of limitation. Any sub-titles herein are included for convenience only, and are not to be construed as limiting the disclosure in any way.

## Claims

1. A peptide that selectively binds αvβ6 integrin, the peptide having an amino acid sequence comprising the motif X₁BₙRGDLX₂X₃X₄ZₘX₅ (SEQ ID NO: 3), wherein X₁ is D-Asn, Bₙ is a sequence of any n number of amino acids, which may be natural or unnatural, D- or L-, and may be the same or different, wherein n is a number between 1 and 10, X₂ and X₃ are independently selected from any amino acid, X₄ is Leu or Ile, Zₘ is a sequence of any m number of amino acids, which may be natural or unnatural, D- or L-, and may be the same or different, wherein m is a number between 1 and 10, and wherein X₅ is any L- or D-amino acid.

2. The peptide according to claim 1, wherein the length of the peptide is between 17 and 25 amino acids, optionally wherein the length of the peptide is 20 amino acids and/or wherein X₅ is L-Thr or D-Thr and/or wherein X₄ is Leu.

3. The peptide according to claim 1 or claim 2, wherein n is 5 and/or m is 6 and/or wherein Bₙ is AVPNL (SEQ ID NO: 4), KVPNL (SEQ ID NO: 5) or K(biotin)VPNL and/or wherein Zₘ is AQKVAR (SEQ ID NO: 6) .

4. The peptide according to any one of the preceding claims, wherein the amino acid sequence of the peptide is selected from the group consisting of:
H₂N-[D-Asn]-AVPNLRGDLQVLAQKVART-COOH (SEQ ID NO: 7);
H₂N-[D-Asn]-KVPNLRGDLQVLAQKVART-COOH (SEQ ID NO: 8);
H₂N-[D-Asn]-K(biotin)VPNLRGDLQVLAQKVART-COOH (SEQ ID NO: 8);
H₂N-[D-Asn]-KVPNLRGDLQVLAQKVAR[D-Thr]-COOH (SEQ ID NO: 9);
H₂N-[D-Asn]-K(biotin)VPNLRGDLQVLAQKVAR[D-Thr]-COOH (SEQ ID NO: 9); and
H₂N-[D-Asn]-AVPNLRGDLQVLAQKVAR[D-Thr]-COOH (SEQ ID NO: 10).

5. The peptide according to any one of the preceding claims, wherein the peptide is N-acetylated and/or C-amidated.

6. A conjugate comprising a peptide as defined in any one of claims 1 to 5 conjugated directly or via a linker to a therapeutic moiety, a polymer, a polypeptide and/or a detectable moiety.

7. The conjugate according to claim 6, wherein the therapeutic moiety comprises an anti-cancer agent, and optionally wherein the anti-cancer agent is selected from the group consisting of: an auristatin, a maytansinoids, a tubulysin, duocarmycin, calicheamicin, alpha-amanitin, a pyrrolobenzodiazepine, irinotecan, and an indolecarboxamide, or a prodrug or active metabolite thereof and optionally, wherein the anti-cancer agent comprises: Monomethyl Auristatin E (MMAE), mertansine (DM1), ravtansine (DM4) or Centanamycin.

8. The conjugate according to either of claims 6 or 7, wherein the therapeutic moiety comprises a radioactive isotope selected from the group consisting of: ¹⁷⁷Lu, ⁹⁰Y, ²¹¹At, ²¹³Bi, ²¹²Pb, ²²⁵Ac, ²²³Ra, ⁴⁴Sc, ⁶⁷Ga, ¹³¹I, ¹⁸⁸Re, ¹⁸⁶Re and ⁶⁷CU.

9. The conjugate according to any one of claims 6 to 8, wherein the detectable moiety is detectable by Magnetic Resonance Imaging (MRI), Magnetic Resonance Spectroscopy (MRS), Single Photon Emission Computed Tomography (SPECT), Positron Emission Tomograph (PET) or optical imaging and optionally, wherein the detectable moiety comprises a fluorophore, a radionuclide or a spin label and optionally, wherein the detectable moiety comprises: ⁶⁸Ga, IRDye® 700, IRDye® 800, Fluorescein isothiocyanate (FITC), ⁸⁹Zr, ¹²⁴I, ⁶⁴Cu, ⁶²Cu, ¹⁸F, ⁸⁶Y, ¹¹¹In, ¹³¹I, ¹²³I, ⁶⁷Ga or ^{99m}Tc.

10. The conjugate according to any one of claims 6 to 9, wherein the detectable moiety comprises ¹¹¹In coupled to the peptide via a linker that comprises a chelator, optionally wherein the chelator comprises diethylenetriamine pentaacetic acid (DTPA), tetrazacyclododecane-1,4,7,10-tetraacetic acid (DOTA) or ethylenediaminetetraacetic acid (EDTA)and/or, wherein the polymer comprises one or more, preferably one or two, ethylene glycol groups or one or more, preferably one or two, polyethylene glycol (PEG) chains and optionally, wherein the one or more, preferably one or two, PEG chains are between 1 and 50 ethylene glycol units in length.

11. A pharmaceutical composition comprising:
a peptide as defined in any one of claims 1 to 5 or a conjugate as defined in any one of claims 6 to 10; and
a pharmaceutically acceptable carrier.

12. A peptide as defined in any one of claims 1 to 5, a conjugate as defined in any one of claims 6 to 10 or a pharmaceutical composition as defined in claim 11 for use in medicine.

13. A peptide as defined in any one of claims 1 to 5, a conjugate as defined in any one of claims 6 to 10 or a pharmaceutical composition as defined in claim 11 for use in a method of treatment of an αvβ6-expressing tumour in a mammalian subject.

14. The peptide, conjugate or composition for use according to claim 13, wherein the tumour is a cervical tumour, a head or neck tumour, a breast tumour, a lung tumour, a skin tumour, a colon tumour, an ovarian tumour or a pancreatic tumour.

15. A conjugate as defined in either of claims 9 or 10 for use in an *in vivo* method of diagnosis of cancer in a mammalian subject, wherein the method comprises administering the conjugate to the subject and detecting said detectable moiety thereby diagnosing the presence of an αvβ6-expressing tumour in the subject.

## Patentansprüche

1. Peptid, das selektiv αvβ6-Integrin bindet, wobei das Peptid eine Aminosäuresequenz, umfassend das Motiv X₁BₙRGDLX₂X₃X₄ZₘX₅ (SEQ ID NO: 3), aufweist, wobei X₁ für D-Asn steht, Bₙ eine Sequenz einer beliebigen Anzahl n von Aminosäuren ist, die natürlich oder künstlich, D- oder L- und gleich oder unterschiedlich sein können, wobei n eine Zahl zwischen 1 und 10 ist, X₂ und X₃ jeweils unabhängig aus einer beliebigen Aminosäure ausgewählt sind, X₄ Leu oder Ile ist, Zₘ eine Sequenz einer beliebigen Anzahl m von Aminosäuren ist, die natürlich oder künstlich, D- oder L- und gleich oder unterschiedlich sein können, wobei m eine Zahl zwischen 1 und 10 ist, und wobei X₅ eine beliebige L- oder D-Aminosäure ist.

2. Peptid nach Anspruch 1, wobei die Länge des Peptids zwischen 17 und 25 Aminosäuren beträgt, wobei die Länge des Peptids gegebenenfalls 20 Aminosäuren beträgt und/oder wobei X₅ für L-Thr oder D-Thr steht und/oder wobei X₄ Leu ist.

3. Peptid nach Anspruch 1 oder Anspruch 2, wobei n = 5 ist und/oder wobei m = 6 ist und/oder wobei Bₙ AVPNL (SEQ ID NO: 4), KVPNL (SEQ ID NO: 5) oder K(biotin)VPNL ist und/oder wobei Zₘ AQKVAR (SEQ ID NO: 6) ist.

4. Peptid nach einem der vorangegangenen Ansprüche, wobei die Aminosäuresequenz des Peptids aus der aus folgenden bestehenden Gruppe ausgewählt ist:
H₂N-[D-Asn]-AVPNLRGDLQVLAQKVART-COOH (SEQ ID NO: 7);
H₂N-[D-Asn]-KVPNLRGDLQVLAQKVART-COOH (SEQ ID NO: 8);
H₂N-[D-Asn]-K(biotin)VPNLRGDLQVLAQKVART-COOH (SEQ ID NO: 8);
H₂N-[D-Asn]-KVPNLRGDLQVLAQKVAR[D-Thr]-COOH (SEQ ID NO: 9);
H₂N-[D-Asn]-K(biotin)VPNLRGDLQVLAQKVAR[D-Thr]-COOH (SEQ ID NO: 9); und
H₂N-[D-Asn]-AVPNLRGDLQVLAQKVAR [D-Thr]-COOH (SEQ ID NO: 10).

5. Peptid nach einem der vorangegangenen Ansprüche, wobei das Peptid N-acetyliert und/oder C-amidiert ist.

6. Konjugat, das ein Peptid nach einem der Ansprüche 1 bis 5 umfasst, das direkt oder über einen Linker an eine therapeutische Gruppierung, ein Polymer, ein Polypeptid und/oder eine detektierbare Gruppierung konjugiert ist.

7. Konjugat nach Anspruch 6, wobei die therapeutische Gruppierung ein Antikrebsmittel umfasst und wobei das Antikrebsmittel gegebenenfalls aus der aus folgenden bestehenden Gruppe ausgewählt ist: Auristatin, Maytansinoiden, Tubulysin, Duocarmycin, Calicheamicin, alpha-Amanitin, Pyrrolobenzodiazepin, Irinotecan und Indolcarboxamid oder einem Prodrug oder aktiven Metaboliten davon, und wobei das Antikrebsmittel gegebenenfalls Monomethylauristatin E (MMAE), Mertansin (DM1), Ravtansin (DM4) oder Centanamycin umfasst.

8. Konjugat nach Anspruch 6 oder 7, wobei die therapeutische Gruppierung ein radioaktives Isotop umfasst, das aus der aus folgenden bestehenden Gruppe ausgewählt ist: ¹⁷⁷Lu, ⁹⁰Y, ²¹¹At, ²¹³Bi, ²¹²Pb, ²²⁵Ac, ²²³Ra, ⁴⁴Sc, ⁶⁷Ga, ¹³¹I, ¹⁸⁸Re, ¹⁸⁶Re und ⁶⁷Cu.

9. Konjugat nach einem der Ansprüche 6 bis 8, wobei die detektierbare Gruppierung mittels Magnetresonanztomographie (MRT), Magnetresonanzspektroskopie (MRS), Einzelphotonen-Emissionscomputertomographie (SPECT), Positronenemissionstomographie (PET) oder optischer Bildgebung detektierbar ist und wobei die detektierbare Gruppierung gegebenenfalls ein Fluorophor, ein Radionuclid oder eine Spinmarkierung umfasst und wobei die detektierbare Gruppierung gegebenenfalls ⁶⁸Ga, IRDye® 700, IRDye® 800, Fluoresceinisothiocyanat (FITC), ⁸⁹Zr, ¹²⁴I, ⁶⁴Cu, ⁶²Cu, ¹⁸F, ⁸⁶Y, ¹¹¹In, ¹³¹I, ¹²³I, ⁶⁷Ga oder ^{99m}Tc umfasst.

10. Konjugat nach einem der Ansprüche 6 bis 9, wobei die detektierbare Gruppierung ¹¹¹In umfasst, das über einen Linker, der einen Chelatbildner umfasst, an das Peptid gebunden ist, wobei der Chelatbildner gegebenenfalls Diethylentriaminpentaessigsäure (DTPA), Tetrazacyclododecan-1,4,7,10-tetraessigsäure (DOTA) oder Ethylendiamintetraessigsäure (EDTA) umfasst und/oder wobei das Polymer eine oder mehrere, vorzugsweise eine oder zwei, Ethylenglykolgruppen oder eine oder mehr, vorzugsweise eine oder zwei, Polyethylenglykol- (PEG-) Ketten umfasst und wobei die eine oder mehreren, vorzugsweise eine oder zwei, PEG-Ketten gegebenenfalls zwischen 1 und 50 Ethylenglykoleinheiten lang sind.

11. Pharmazeutische Zusammensetzung, die ein Peptid nach einem der Ansprüche 1 bis 5 oder ein Konjugat nach einem der Ansprüche 6 bis 10 und einen pharmazeutisch annehmbaren Träger umfasst.

12. Peptid nach einem der Ansprüche 1 bis 5, Konjugat nach einem der Ansprüche 6 bis 10 oder pharmazeutische Zusammensetzung nach Anspruch 11 zur Verwendung in der Medizin.

13. Peptid nach einem der Ansprüche 1 bis 5, Konjugat nach einem der Ansprüche 6 bis 10 oder pharmazeutische Zusammensetzung nach Anspruch 11 zur Verwendung in einem Verfahren zur Behandlung eines αvβ6-exprimierenden Tumors bei einem Säugetierindividuum.

14. Peptid, Konjugat oder Zusammensetzung zur Verwendung nach Anspruch 13, wobei der Tumor ein Gebärmutterhalstumor, Kopf- oder Halstumor, Brusttumor, Lungentumor, Hauttumor, Dickdarmtumor, Eierstocktumor oder Bauchspeicheldrüsentumor ist.

15. Konjugat nach Anspruch 9 oder 10 zur Verwendung in einem In-vivo-Verfahren zur Diagnose von Krebs bei einem Säugetierindividuum, wobei das Verfahren das Verabreichen des Konjugats an das Individuum und das Detektieren der detektierbaren Gruppierung umfasst, wodurch die Gegenwart eines αvβ6-exprimierenden Tumors bei dem Individuum diagnostiziert wird.

## Revendications

1. Peptide qui se lie sélectivement à une intégrine αvβ6, le peptide ayant une séquence d'acides aminés comprenant le motif X₁BₙRGDLX₂X₃X₄ZₘX₅ (SEQ ID NO : 3), dans lequel X₁ est D-Asn, Bₙ est une séquence d'un quelconque nombre n d'acides aminés, qui peuvent être naturels ou non-naturels, D- ou L-, et peuvent être identiques ou différents, dans lequel n est un nombre compris entre 1 et 10, X₂ et X₃ sont indépendamment choisis parmi tout acide aminé, X₄ est Leu ou Ile, Zₘ est une séquence d'un quelconque nombre m d'acides aminés, qui peuvent être naturels ou non-naturels, D- ou L-, et peuvent être identiques ou différents, dans lequel m est un nombre compris entre 1 et 10, et dans lequel X₅ est tout acide L- ou D-aminé.

2. Peptide selon la revendication 1, dans lequel la longueur du peptide est comprise entre 17 et 25 acides aminés, facultativement dans lequel la longueur du peptide est de 20 acides aminés et/ou dans lequel X₅ est L-Thr ou D-Thr et/ou dans lequel X₄ est Leu.

3. Peptide selon la revendication 1 ou la revendication 2, dans lequel n est égal à 5 et/ou m est égal à 6 et/ou dans lequel Bₙ est AVPNL (SEQ ID NO : 4), KVPNL (SEQ ID NO : 5) ou K(biotine)VPNL et/ou dans lequel Zₘ est AQKVAR (SEQ ID NO : 6).

4. Peptide selon l'une quelconque des revendications précédentes, dans lequel la séquence d'acides aminés du peptide est choisie parmi le groupe constitué de :
H₂N-[D-Asn]-AVPNLRGDLQVLAQKVART-COOH (SEQ ID NO : 7) ;
H₂N-[D-Asn]-KVPNLRGDLQVLAQKVART-COOH (SEQ ID NO : 8) ;
H₂N-[D-Asn]-K(biotine)VPNLRGDLQVLAQKVART-COOH (SEQ ID NO : 8) ;
H₂N-[D-Asn]-KVPNLRGDLQVLAQKVAR[D-Thr]-COOH (SEQ ID NO : 9) ;
H₂N-[D-Asn]-K(biotine)VPNLRGDLQVLAQKVAR[D-Thr]-COOH (SEQ ID NO : 9) ; et
H₂N-[D-Asn]-AVPNLRGDLQVLAQKVAR[D-Thr]-COOH (SEQ ID NO : 10).

5. Peptide selon l'une quelconque des revendications précédentes, dans lequel le peptide est N-acétylé et/ou C-amidé.

6. Conjugué comprenant un peptide tel que défini dans l'une quelconque des revendications 1 à 5 conjugué directement ou via un lieur à un fragment thérapeutique, un polymère, un polypeptide et/ou un fragment détectable.

7. Conjugué selon la revendication 6, dans lequel le fragment thérapeutique comprend un agent anticancéreux, et facultativement dans lequel l'agent anticancéreux est choisi parmi le groupe constitué de : auristatine, maytansinoïdes, tubulysine, duocarmycine, calichéamicine, alpha-amanitine, pyrrolobenzodiazépine, irinotécan, et indolécarboxamide, ou pro-médicament ou métabolite actif de ceux-ci et facultativement, dans lequel l'agent anticancéreux comprend : Monométhyle Auristatine E (MMAE), mertansine (DM1), ravtansine (DM4) ou Centanamycine.

8. Conjugué selon l'une des revendications 6 ou 7, dans lequel le fragment thérapeutique comprend un isotope radioactif choisi parmi le groupe constitué de : ¹⁷⁷Lu, ⁹⁰Y, ²¹¹At, ²¹³Bi, ²¹²Pb, ²²⁵Ac, ²²³Ra, ⁴⁴Sc, ⁶⁷Ga, ¹³¹I, ¹⁸⁸Re, ¹⁸⁶Re et ⁶⁷Cu.

9. Conjugué selon l'une quelconque des revendications 6 à 8, dans lequel le fragment détectable est détectable par Imagerie par Résonance Magnétique (IRM), Spectroscopie par Résonance Magnétique (MRS), Tomographie par Émission Monophotonique (SPECT), Tomographie par Émission de Positrons (PET) ou imagerie optique et facultativement, dans lequel le fragment détectable comprend un fluorophore, un radionucléide ou un marqueur de spin et facultativement, dans lequel le fragment détectable comprend : ⁶⁸Ga, IRDye® 700, IRDye® 800, Isothiocyanate de fluorescéine (FITC), ⁸⁹Zr, ¹²⁴I, ⁶⁴Cu, ⁶²Cu, ¹⁸F, ⁸⁶Y, ¹¹¹In, ¹³¹I, ¹²³I, ⁶⁷Ga ou ^{99m}Tc.

10. Conjugué selon l'une quelconque des revendications 6 à 9, dans lequel le fragment détectable comprend ¹¹¹In couplé au peptide via un lieur qui comprend un chélateur, facultativement dans lequel le chélateur comprend de l'acide diéthylènetriamine pentaacétique (DTPA), de l'acide tétrazacyclododécane-1,4,7,10-tétraacétique (DOTA) ou de l'acide éthylènediaminetétraacétique (EDTA) et/ou, dans lequel le polymère comprend un ou plusieurs, de préférence un ou deux, groupes éthylène glycol ou une ou plusieurs, de préférence une ou deux, chaînes polyéthylène glycol (PEG) et facultativement, dans lequel les une ou plusieurs, de préférence une ou deux, chaînes PEG ont une longueur comprise entre 1 et 50 unités d'éthylène glycol.

11. Composition pharmaceutique comprenant :
un peptide tel que défini dans l'une quelconque des revendications 1 à 5 ou un conjugué tel que défini dans l'une quelconque des revendications 6 à 10 ; et
un support pharmaceutiquement acceptable.

12. Peptide tel que défini dans l'une quelconque des revendications 1 à 5, conjugué tel que défini dans l'une quelconque des revendications 6 à 10 ou composition pharmaceutique telle que définie dans la revendication 11 pour une utilisation en médecine.

13. Peptide tel que défini dans l'une quelconque des revendications 1 à 5, conjugué tel que défini dans l'une quelconque des revendications 6 à 10 ou composition pharmaceutique telle que définie dans la revendication 11 à utiliser dans un procédé de traitement d'une tumeur exprimant αvβ6 chez un sujet mammifère.

14. Peptide, conjugué ou composition à utiliser selon la revendication 13, dans lequel la tumeur est une tumeur du col de l'utérus, une tumeur de la tête ou du cou, une tumeur du sein, une tumeur du poumon, une tumeur de la peau, une tumeur du côlon, une tumeur des ovaires ou une tumeur du pancréas.

15. Conjugué tel que défini dans l'une quelconque des revendications 9 ou 10 à utiliser dans un procédé *in vivo* de diagnostic de cancer chez un sujet mammifère, dans lequel le procédé comprend l'administration du conjugué au sujet et la détection dudit fragment détectable diagnostiquant ainsi la présence d'une tumeur exprimant αvβ6 chez le sujet.
